# EUROPEAN PATENT SPECIFICATION

(11) **EP 3 323 396 B1**
(45) Date of publication and mention of the grant of the patent: **31.03.2021**
(21) Application number: 16824280.8
(22) Date of filing: 30.06.2016
(51) Int. Cl.: A61F 13/49, A61F 13/15, A61F 13/496

(54) **DISPOSABLE UNDERGARMENT AND METHOD FOR PRODUCING DISPOSABLE UNDERGARMENT**
EINWEGUNTERWÄSCHE UND VERFAHREN ZUR HERSTELLUNG VON EINWEGUNTERWÄSCHE
SOUS-VÊTEMENT JETABLE ET PROCÉDÉ DE FABRICATION DE SOUS-VÊTEMENT JETABLE

(30) Priority: 13.07.2015 JP 2015140097; 13.07.2015 JP 2015140096
(43) Date of publication of application: 23.05.2018
(73) Proprietor: Koyo Corporation, Kanagawa 236-0004 (JP)
(72) Inventor: SHIRAI Atsuko, Yokohama-shi Kanagawa 236-0004 (JP); MATSUMIYA Munetada, Yokohama-shi Kanagawa 236-0004 (JP); MITUGI Takehisa, Yokohama-shi Kanagawa 236-0004 (JP); ISE Kazuya, Yokohama-shi Kanagawa 236-0004 (JP)
(74) Representative: Samson & Partner Patentanwälte mbB
(86) International application number: PCT/JP2016/069406
(87) International publication number: WO 2017/010300

(56) References cited:
- EP-A1- 1 473 009
- WO-A1-2004/052260
- WO-A1-2013/018807
- JP-A- H07 213 553
- JP-A- 2003 305 082
- JP-A- 2005 198 784
- JP-A- 2006 043 068
- JP-A- 2009 528 888
- JP-A- 2010 110 536
- JP-A- 2010 227 654
- JP-A- 2012 192 115
- JP-A- 2013 031 536

## Description

### TECHNICAL FIELD

The present invention relates to a disposable undergarment that is particularly a pant-type diaper and pad-holder, and to a method for manufacturing the disposable undergarment.

### BACKGROUND ART

Conventionally known disposable undergarments, such as a pant-type disposable diaper and pad holder, have a long rise length. When a normal wearer wears the undergarment, a waist opening is positioned substantially horizontally around the waist of the wearer. The waist opening includes an elastic member that is stretched and contracted in a waist-around direction. For example, Patent Document 1 discloses a pant-type disposable diaper having the following feature. Specifically, when a user wearing the pant-type disposable diaper and sits on a floor, a waist opening is positioned substantially horizontally around the waist of the wearer in side view.

### RELATED ART DOCUMENTS

### PATENT DOCUMENTS

Patent Document 1: Japanese Patent No. 3916878

The disposable diaper with the waist opening positioned substantially horizontally around the waist of the wearer is supported at a wearing position with the waist opening compressing the waist with no bone. Logically, the diaper largely deforms when the wearer sits down or bends over, and thus is likely to be shifted from the wearing position. The wearer taking such a posture might feel uncomfortable with the diaper compressing the stomach area. Furthermore, the disposable diaper has the long rise length and thus is likely to stick out from pants and skirts. The disposable diaper is made of nonwoven fabric and thus has lower flexibility and absorbency than an undergarment made of cloth. Thus, the disposable diaper with a large area covering the wearer cannot be comfortably worn. Such a diaper is particularly unsuitable for relatively active adults.

WO 2013/018807 A1 relates to a disposable wearing article such as a pant-type disposable diaper having a waist elastic body.

### SUMMARY OF THE INVENTION

### PROBLEMS TO BE SOLVED BY THE INVENTION

An object of the present invention is to provide a disposable undergarment that has a short rise length, does not excessively compress the waist of a wearer, and achieves a stable wearing position. Another object of the present invention is to provide an appropriate method for manufacturing the disposable undergarment.

### MEANS OF SOLVING THE PROBLEMS

To solve the problem described above, a disposable undergarment according to the present invention is defined in independent claim 1.

A method for manufacturing the disposable undergarment according to the present invention is defined in independent claim 7.

Preferred embodiments are defined in the dependent claims.

Further details, features, and advantages of the present invention will be apparent from the description on preferred exemplary embodiments given with reference to the figures.

The embodiments or examples of the following description which are not covered by the appended claims are considered as not being part of the present invention according to this description.

### BRIEF DESCRIPTION OF THE DRAWINGS

FIG. 1 is a front view of a disposable undergarment according to one embodiment of the present invention.
FIG. 2 is a back view of the disposable undergarment.
FIG. 3 is a diagram illustrating a planned cutting line for forming a ventral member and a dorsal member of the disposable undergarment, elastic members in a stretched state, and non-elastic areas.
FIG. 4 is a diagram illustrating the disposable undergarment in an open state with the elastic member in a stretched state.
FIG. 5 is a front view of a crotch member of the disposable undergarment in a state where elastic members in a crotch member are stretched.
FIG. 6 is a schematic cross-sectional view of the crotch member illustrated in FIG. 5 taken along line VI-VI.
FIG. 7 is a front view illustrating a state where the disposable undergarment is worn.
FIG. 8 is a back view illustrating the state where the disposable undergarment is worn.
FIG. 9 is a left side view illustrating the state where the disposable undergarment is worn.
FIG. 10 is a diagram illustrating manufacturing steps for a disposable undergarment according to one embodiment of the present invention.
FIG. 11 is a diagram illustrating a disposable undergarment according to another embodiment in an open state with elastic members in a stretched state.
FIG. 12 is a diagram illustrating a disposable undergarment according to still another embodiment in an open state with elastic members in a stretched state.
FIG. 13 is a diagram illustrating a disposable undergarment according to yet still another embodiment in an open state with elastic members in a stretched state.

### MODE FOR CARRYING OUT THE INVENTION

One exemplary embodiment of the present invention is described below.

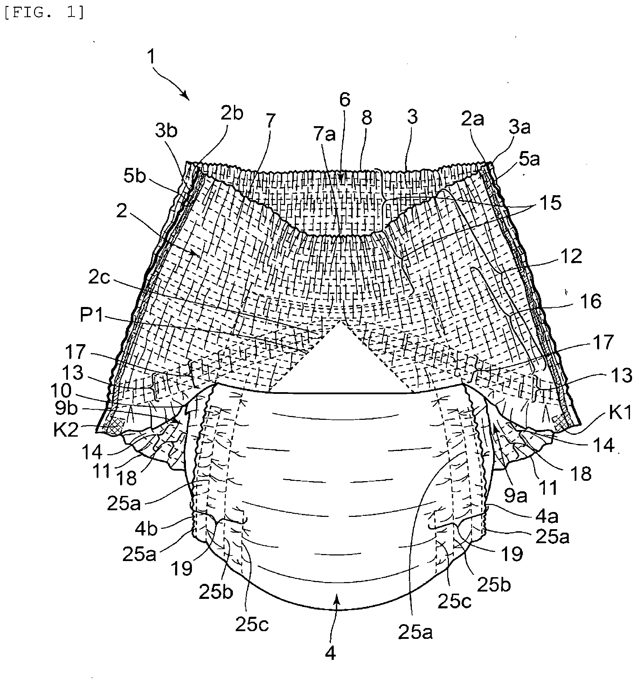

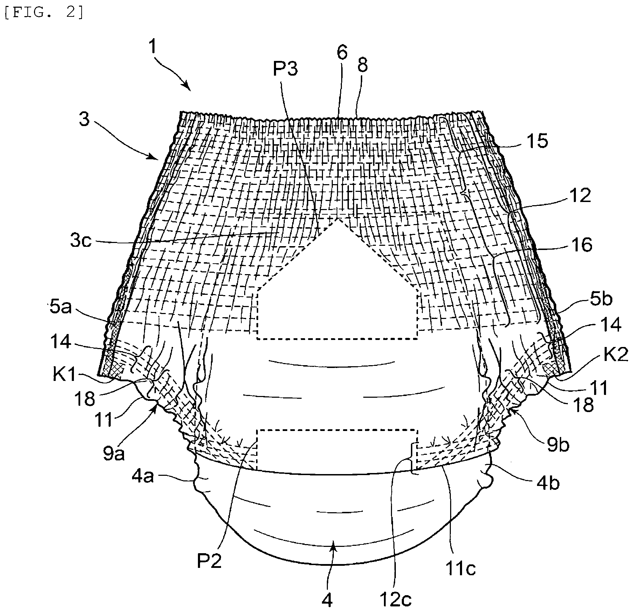

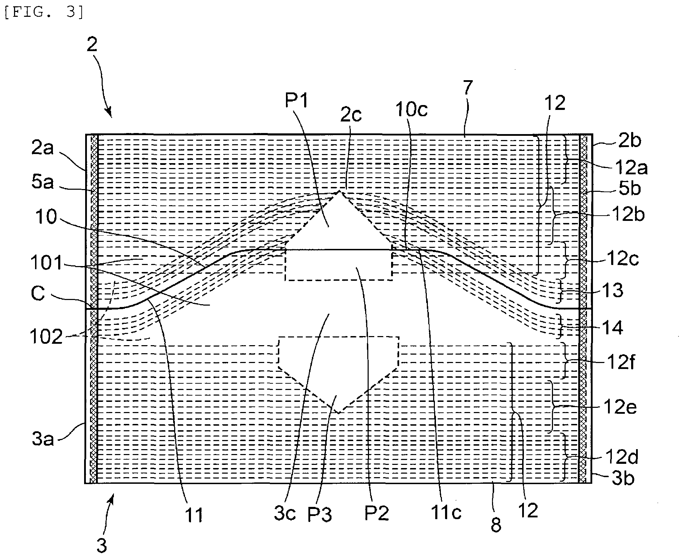

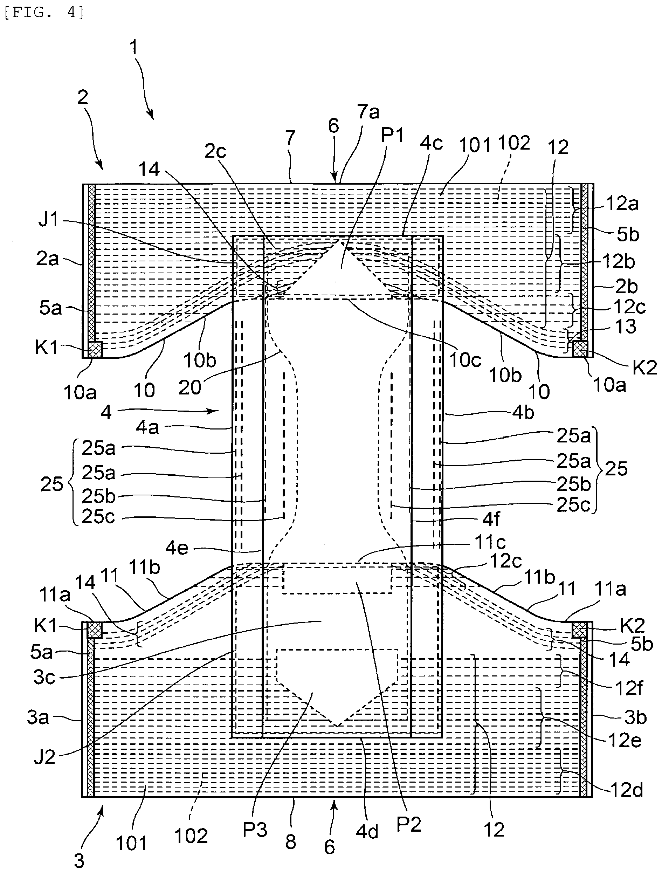

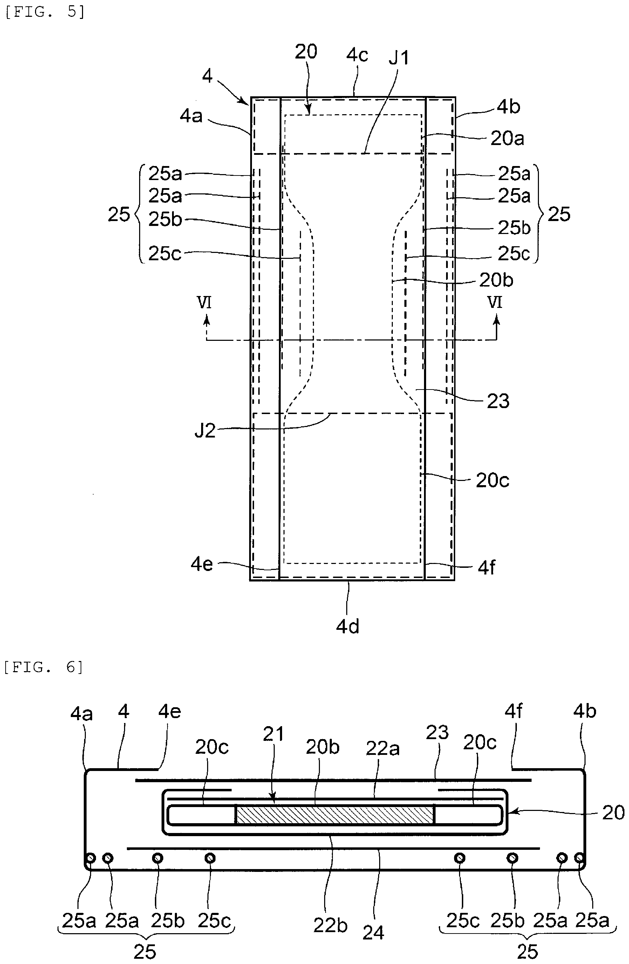

FIG. 1 is a front view of a pant-type disposable diaper 1 as a disposable undergarment according to one exemplary embodiment of the present invention. FIG. 2 is a back view of the disposable diaper 1. FIG. 3 is a diagram illustrating a planned cutting line for forming a ventral member 2 and a dorsal member 3 of the disposable diaper 1, elastic members in a stretched state, and non-elastic areas. FIG. 4 is a diagram illustrating the disposable diaper 1 in an open state with the elastic member in a stretched state. FIG. 5 is a front view of the disposable diaper 1 in a state where elastic members in a crotch member 4 are stretched. FIG. 6 is a schematic cross-sectional view of the crotch member 4 illustrated in FIG. 5 taken along line VI-VI.

As illustrated in FIG. 1 and FIG. 2, the disposable diaper 1 according to the present embodiment includes: the ventral member 2 and the dorsal member 3 respectively disposed on the wearer's ventral and dorsal sides when the disposable diaper 1 is worn; and the crotch member 4 that bridges between and is bonded to center portions 2c and 3c of the members described above. The ventral member 2 and the dorsal member 3 are bonded to each other at both bonded portions 5a and 5b. The bonded portions 5a and 5b are formed along both end portions 2a and 2b of the ventral member 2 in a waist-around direction and along both end portions 3a and 3b of the dorsal member 3 in the waist-around direction. Positioning bonded portions K1 and K2 are formed in lower portions of both bonded portions 5a and 5b and a periphery of the lower portions. A waist opening 6 is defined by an upper edge 7 of the ventral member 2 and an upper edge 8 of the dorsal member 3. Left and right leg openings 9a and 9b are each defined by a lower edge 10 of the ventral member 2 or a lower edge 11 of the dorsal member 3 and by a left side edge 4a or a right side edge 4b of the crotch member 4.

As illustrated in FIG. 1 and FIG. 2, a hip-surrounding elastic member 12 extends in the waist-around direction in each of the ventral member 2 and the dorsal member 3. A first leg-surrounding elastic member 13 as a first elastic member extends in the left and the right leg openings 9a and 9b of the ventral member 2. A second leg-surrounding elastic member 14 extends in the left and the right leg openings 9a and 9b of the dorsal member 3. The elastic members 12, 13, and 14 each lose tensile force in each of non-elastic areas P1, P2, and P3. Waist-surrounding gathers 15 and 15 are formed at and around the waist opening 6 of the ventral member 2 and the dorsal member 3. Hip-surrounding gathers 16 and 16 are formed below the waist-surrounding gathers 15 and 15 of the ventral member 2 and the dorsal member 3. Leg-surrounding gathers 17 and 17 are formed in the left and the right leg openings 9a and 9b of the ventral member 2. Leg-surrounding gathers 18 and 18 are formed in the left and the right leg openings 9a and 9b of the dorsal member 3. Crotch gathers 19 and 19 are formed in an area at and around the left and the right leg openings 9a and 9b of the crotch member 4 in which a crotch elastic member 25 (that is, crotch elastic members 25a, 25b, and 25c) extends.

As illustrated in FIG. 3, the ventral member 2 and the dorsal member 3 of the disposable diaper 1 are formed by cutting a layered body along a planned cutting line C. The layered body has a structure in which an inner sheet 101 positioned on the wearer's side when the diaper is worn and an outer sheet 102 positioned on a side opposite to the wearer are attached to each other with the hip-surrounding elastic member 12, the first leg-surrounding elastic member 13, and the second leg-surrounding elastic member 14 appropriately disposed in between in a stretched state. The lower edge 10 of the ventral member 2 and the lower edge 11 of the dorsal member 3 are formed as a result of the cutting along the common planned cutting line C, and match when being brought into contact with each other in the stretched state.

A material of the inner sheet 101 and the outer sheet 102 of the disposable diaper 1 is a nonwoven fabric including: chemical fiber such as polyethylene, polypropylene, polyester, nylon, and rayon; natural fiber such as cotton and cellulose; or a combination of these. The material is not limited to the nonwoven fabric and may be a resin film, tissues, an elastic material, a layered body including these, or a sheet member formed by appropriately connecting these.

As illustrated in FIG. 4, the ventral member 2 in the stretched state has a rectangular shape with a substantially trapezoidal inward recess on a portion of one longitudinal side not including both ends. Both end portions 2a and 2b in the waist-around direction and the upper edge 7 of the ventral member 2 are linearly formed. The lower edge 10 of the ventral member 2 includes a pair of both end lines 10a and 10a, a pair of inclined lines 10b and 10b, and a center line 10c. Both end lines 10a and 10a are substantially in parallel with the waist-around direction of the ventral member 2, and extend from both end portions 2a and 2b to positions slightly beyond the bonded portions 5a and 5b. The inclined lines 10b and 10b extend gradually upward toward the upper edge 7 from both end lines 10a and 10a to left and right boundaries of the center portion 2c as an area of the ventral member 2 that overlaps with the crotch member 4. The center line 10c is substantially in parallel with the waist-around direction and connects between both inclined lines 10b and 10b.

As illustrated in FIG. 4, the dorsal member 3 in the stretched state has a rectangular shape in which a portion in one long side, not including both ends, protrudes outward in a substantially trapezoidal form. Both end portions 3a and 3b in the waist-around direction and the upper edge 8 of the dorsal member 3 are linearly formed. The lower edge 11 of the dorsal member 3 includes: a pair of both end lines 11a and 11a, a pair of inclined lines 11b and 11b, and a center line 11c. Both end lines 11a and 11a are substantially in parallel with the waist-around direction of the dorsal member 3, and extend from both end portions 3a and 3b to positions slightly beyond the bonded portions 5a and 5b. The inclined lines 11b and 11b gradually extend downward (upward in FIG. 4) toward a side opposite to the upper edge 8 from both end lines 11a and 11a to the left and right boundaries of the center portion 3c as an area of the dorsal member 3 that overlaps with the crotch member 4. The center line 11c is substantially in parallel with the waist-around direction and connects between both inclined lines 11b and 11b.

As illustrated in FIG. 4, the length in the waist-around direction or a left and right direction of the ventral member 2, that is, the length of the linear upper edge 7 of the ventral member 2 is substantially the same as a length of the waist-around direction or the left and right direction of the dorsal member 3, that is, the length of the linear upper edge 8 of the dorsal member 3. A rise length in the ventral member 2, that is, the distance between the upper edge 7 and the lower edge 10 in the ventral member 2 is the longest at a portion around both bonded portions 5a and 5b, and is the shortest at a portion around the left and right boundaries of the center portion 2c that overlaps with the crotch member 4. The rise length in the dorsal member 3, that is, the distance between the upper edge 8 and the lower edge 11 in the dorsal member 3 is the shortest at the portion around both bonded portions 5a and 5b and is the longest at the portion around the left and right boundaries of the center portion 3c that overlaps with the crotch member 4. Both end portions 2a and 2b of the ventral member 2, both end portions 3a and 3b of the dorsal member 3, and the bonded portions 5a and 5b of both members have substantially the same rise length. The difference between the rise length of the bonded portions 5a and 5b of the ventral member 2 and the rise length at the left and right boundaries of the center portion 2c of the ventral member 2, and the difference between the rise length at the left and right boundaries of the center portion 3c of the dorsal member 3 and the rise length of the bonded portions 5a and 5b of the dorsal member 3 are 3 to 12 cm and is more preferably, 5 to 9 cm in disposable diapers for adults.

As illustrated in FIG. 4, the crotch member 4 is bonded to the inner sheets 101 and 101 of the center portions 2c and the 3c of the ventral member 2 and the dorsal member 3. The crotch member 4 is folded in two with the inner sheet 101 disposed on the inner side of the disposable diaper 1, so that both end portions 2a and 2b of the ventral member 2 respectively overlap with both end portions 3a and 3b of the dorsal member 3. The bonded portions 5a and 5b are formed along both end portions 2a and 3a and 2b and 3b overlapped with each other. The bonded portions 5a and 5b can be formed by bonding using adhesive or various sealing methods including heat sealing and ultrasonic sealing. The bonded portions 5a and 5b are formed in areas between the upper edges 7 and 8 and both end lines 10a, 10b, 11a, and 11b of the lower edges in parallel with each other, in the ventral member 2 and the dorsal member 3. Thus, the bonded portions 5a and 5b can be formed along the entire length in the upper and lower direction of both end portions 2a, 2b, 3a, and 3b and to have a sufficient width. The bonded portions 5a and 5b are intermittently formed over the entire length of both end portions 2a, 2b, 3a, and 3b in the upper and lower direction. However, this should not be construed in a limiting sense, and the bonded portions 5a and 5b may be continuously formed over the entire length of both end portions 2a, 2b, 3a, and 3b in the upper and lower direction.

As illustrated in FIG. 4, the positioning bonded portions K1 and K2 are formed to partially overlap with the bonded portions 5a and 5b, in the lower portions of the bonded portions 5a and 5b formed along both end portions 2a, 3a, 2b, and 3b of the ventral member 2 and the dorsal member 3 in the waist-around direction and in a periphery of the lower portions. The positioning bonded portion K1 is not in contact with the end portions 2a and 3a. The positioning bonded portion K2 is not in contact with the end portions 2b and 3b. However, this should not be construed in a limiting sense, and the positioning bonded portions may be in contact with the end portions. The positioning bonded portions may not overlap with the bonded portions 5a and 5b and may be formed as a part of the bonded portions 5a and 5b. The positioning bonded portions may not overlap with the bonded portions 5a and 5b and may be formed in a periphery of the bonded portions 5a and 5b.

As illustrated in FIG. 3 and FIG. 4, the hip-surrounding elastic member 12, the first leg-surrounding elastic member 13 as the first elastic member, and the second leg-surrounding elastic member 14 are each a group of a plurality of elastic members in a thread-like form. The elastic members in a thread-like form are appropriately disposed and bonded on the inner sheets 101 and the outer sheets 102 of the ventral member 2 and the dorsal member 3, while being in the stretched state. The sheets 101 and 102 are bonded to each other with the elastic members disposed in between.

As illustrated in FIG. 3 and FIG. 4, the hip-surrounding elastic member 12 includes hip-surrounding elastic members 12a, 12b, 12c, 12d, 12e, and 12f. The hip-surrounding elastic members 12a, 12b, and 12c are disposed in the ventral member 2 while being stretched in the waist-around direction. The hip-surrounding elastic member 12a is disposed in an area at and around the waist opening 6 along the upper edge 7 of the ventral member 2. The hip-surrounding elastic member 12b is disposed in an area below the hip-surrounding elastic member 12a and above the center line 10c of the ventral member 2. The hip-surrounding elastic member 12c is disposed in an area below the hip-surrounding elastic member 12b and above both end portions of the first leg-surrounding elastic member 13. As illustrated in FIG. 3, the hip-surrounding elastic member 12c crosses the planned cutting line C. Thus, a part of the hip-surrounding elastic member 12c extends in a periphery of the center line 11c of the dorsal member 3.

As illustrated in FIG. 3 and FIG. 4, the hip-surrounding elastic members 12d, 12e, and 12f are disposed in the dorsal member 3 while being stretched in the waist-around direction. The hip-surrounding elastic member 12d is disposed in an area at and around the waist opening 6 along the upper edge 8 of the dorsal member 3. The hip-surrounding elastic member 12e is disposed in an area below the hip-surrounding elastic member 12d, that is, area closer to the lower edge 11 than the hip-surrounding elastic member 12d. The hip-surrounding elastic member 12f is disposed in an area below the hip-surrounding elastic member 12e, that is, area closer to the lower edge 11 than the hip-surrounding elastic member 12e and above the second leg-surrounding elastic member 14, that is, and area closer to the upper edge 8 than the second leg-surrounding elastic member 14. The upper and lower direction length, that is, the length in a direction orthogonal to the waist-around direction of each the areas of the dorsal member 3 respectively including the hip-surrounding elastic members 12d, 12e, and 12f is substantially the same as the upper and lower direction length of a corresponding one of the areas of the ventral member 2 respectively including the hip-surrounding elastic members 12a, 12b, and 12c.

An arrangement interval of the elastic members in the thread-like form, as components of each of the hip-surrounding elastic members 12a, 12b, and 12c in the ventral member 2, is the shortest in the hip-surrounding elastic member 12a, is the second shortest in the hip-surrounding elastic member 12b, and is the longest in the hip-surrounding elastic member 12c. The arrangement interval in each of the hip-surrounding elastic members 12d, 12e, and 12f in the dorsal member 3 is substantially the same as that in a corresponding one of the hip-surrounding elastic members 12a, 12b, and 12c in the ventral member 2. In the present embodiment, the elastic members in the thread-like form as the components of the hip-surrounding elastic member 12a are arranged at an equal arrangement interval. However, this should not be construed in a limiting sense, and the elastic members may not be arranged at an equal interval. The same applies to the other hip-surrounding elastic members 12b to 12f. Thus, the arrangement interval in the hip-surrounding elastic member 12 is not limited to that described above, and may be adjusted as appropriate to facilitate an attempt to achieve better fitting and outer appearance based on the shape of each portion of the wearer's body.

In the present embodiment, the hip-surrounding elastic members 12a to 12c of the ventral member 2, the first leg-surrounding elastic member 13, and the second leg-surrounding elastic member 14 have the same tensile force and stretching ratio per unit length. The hip-surrounding elastic members 12d to 12f of the dorsal member 3 have a lower stretching ratio than the hip-surrounding elastic members 12a to 12c of the ventral member 2. In other words, the hip-surrounding elastic member 12 in the ventral member 2 has higher tensile force than the hip-surrounding elastic member 12 in the dorsal member 3. The ventral member 2 and the dorsal member 3 have substantially the same length in the waist-around direction. As illustrated in FIG. 1, the ventral member 2 contracts more in the waist-around direction than the dorsal member 3, and thus the bonded portions 5a and 5b appear on the side of the ventral member 2 of the disposable diaper 1, that is, on the front side.

As a modification of the present embodiment, in the ventral member 2, the tensile force of the hip-surrounding elastic member 12 may increase in the order of the hip-surrounding elastic members 12a, 12b, and 12c. Furthermore, in the dorsal member 3, the tensile force of the hip-surrounding elastic member 12 may increase in the order of the hip-surrounding elastic members 12d, 12e, and 12f. In this manner, the tensile force of the hip-surrounding elastic members 12 in the ventral member 2 and the dorsal member 3 may decrease from the waist opening 6 toward the leg openings 9a and 9b on the lower side, on an area-to-area basis or in a stepwise manner. With the disposable diaper 1 according to this modification, the fitting can be improved for the wearer through more detailed adjustment on the shape widening from the waist opening 6 toward the leg openings 9a and 9b on the lower side due to the contraction of the waist opening 6. The tensile force of the hip-surrounding elastic member 12 may gradually decrease from the waist opening 2 toward the leg openings 9a and 9b on the lower side not on an area-to-area basis, but on an elastic member-to-elastic member basis. The elastic members may be different from each other in the tensile force by having different stretching ratios or different elastic moduli.

As illustrated in FIG. 3 and FIG. 4, the first leg-surrounding elastic member 13 as the first elastic member does not extend along the lower edge 10 defining the left and the right leg openings 9a and 9b of the ventral member 2. Instead, the first leg-surrounding elastic member 13 extends upward toward the upper edge 7 from lower portions of both bonded portions 5a and 5b to the center of the ventral member 2 in the waist-around direction. The first leg-surrounding elastic member 13 extends substantially linearly in a direction from the lower portions of the bonded portions 5a and 5b toward the center of a portion around the lower end of the hip-surrounding elastic member 12a. Excellent fitting can be achieved for the wearer in the area where the first leg-surrounding elastic member 13 extends, that is, the area of the ventral member 2 that overlaps with the crotch member 4 and an area of the leg openings 9a and 9b in a periphery thereof. The first leg-surrounding elastic member 13 has a center portion in a gently curved form. The tensile force of the first leg-surrounding elastic member 13 acts in such a manner that the first leg-surrounding elastic member 13 linearly extends from the lower portion of the left bonded portion 5a to the lower portion of the right bonded portion 5b. Thus, force of pulling down a center portion 7a of the upper edge 7 of the ventral member 2 is generated. As a result, the disposable undergarment 1 has the center portion 7a of the upper edge 7 of the ventral member 2 positioned lower than the upper edge 8 of the dorsal member 3. The elastic members in a thread-like form in the first leg-surrounding elastic member 13 are arranged in parallel at an equal arrangement interval. However, this should not be construed in a limiting sense. The elastic members may not be arranged in parallel or may not be arranged at an equal arrangement interval.

As illustrated in FIG. 3 and FIG. 4, the second leg-surrounding elastic member 14 extends downward toward the side opposite to the upper edge 8 from the lower portions of both bonded portions 5a and 5b of the dorsal member 3 to the center line 11c. The second leg-surrounding elastic member 14 extends substantially linearly from the lower portions of the bonded portions 5a and 5b to the left and right end portions of the center line 11c. Excellent fitting can be achieved for the wearer in an area of the dorsal member 3 in which the second leg-surrounding elastic member 14 extends. The second leg-surrounding elastic member 14 crosses the hip-surrounding elastic member 12c in an area including the center portion 3c of the dorsal member 3 that overlaps with the crotch member 4 and peripheral areas on the left and right sides of the center portion 3c. As illustrated in FIG. 3, the second leg-surrounding elastic member 14 crosses the planned cutting line C. Thus, a part of the second leg-surrounding elastic member 14 extends in the area of the center portion 2c of the ventral member 2. The elastic members in a thread-like form in the second leg-surrounding elastic member 14 are arranged in parallel at an equal arrangement interval. However, this should not be construed in a limiting sense. The elastic members may not be arranged in parallel or may not be arranged at an equal arrangement interval.

The elastic members in a thread-like form in the hip-surrounding elastic member 12, the first leg-surrounding elastic member 13, and the second leg-surrounding elastic member 14 are each made of a flexible material such as natural rubber, polyurethane resin, and flexible hot melt. The elastic members 12, 13, and 14 are not limited to the elastic member in a thread-like form. Elastic members in a strip form or a sheet form may be employed. The ventral member 2 or the dorsal member 3 may be entirely made of a material such as a flexible sheet. When the elastic members in the sheet form are used or when the ventral member 2 or the dorsal member 3 is entirely made of the flexible sheet, the tensile force acting in the extending direction of the elastic members 12, 13, and 14 is required.

In FIG. 3 and FIG. 4, the elastic members lose their tensile force in the non-elastic areas P1, P2, and P3. For example, processes including providing multiple protrusions, cutting the elastic member with a cutter blade or the like, heat sealing, and the like may be appropriately selected for a process with which the elastic member lose the tensile force.

As illustrated in FIG. 3 and FIG. 4, the non-elastic area P1 is disposed at the center of the ventral member 2 in the waist-around direction, and has a triangular shape with the apex on the side of the upper edge 7. The apex of the non-elastic area P1 having the triangular shape is on a center line of the ventral member 2 in the waist-around direction, and is positioned at a portion around an upper end of the area including the hip-surrounding elastic member 12b. The bottom line of the non-elastic area P1 having the triangular shape is positioned on the center line 10c of the lower edge 10. With the non-elastic area P1 formed in the manner described above, the hip-surrounding elastic member 12b is divided at the center portion in such a manner that a portion without the tensile force becomes longer toward the lower edge 10.

The shape of the non-elastic area P1 is not limited to a triangle, and may be any tapered shape with the apex on the side of the upper edge 7 of the ventral member 2. The example of such a shape includes a parabola or arch shape.

As illustrated in FIG. 3 and FIG. 4, the hip-surrounding elastic member 12a in the ventral member 2 has no non-elastic area formed, and thus has the longest portion with the tensile force. The hip-surrounding elastic member 12b is divided into left and right parts by the non-elastic area P1 in such a manner that a portion with the tensile force becomes shorter toward the lower edge 10. The hip-surrounding elastic member 12c is divided into left and right parts by the lower edge 10 to have a portion with the tensile force that is shorter than that of the hip-surrounding elastic member 12b. The portion with the tensile force becomes shorter toward the lower side.

With the non-elastic area P1 formed in the manner described above, the ventral member 2 has the portion with the tensile force in the hip-surrounding elastic member 12 that becomes shorter from the waist opening 6 toward the leg openings 9a and 9b on the lower side. Thus, as illustrated in FIG. 1, the ventral member 2 has a shape gently widening from the waist opening 6 toward the leg openings 9a and 9b on the lower side due to the contraction of the waist opening 6. Thus, the disposable diaper 1 can achieve excellent fitting for the wearer with a general body shape widening from the waist toward the upper ends of both legs.

As illustrated in FIG. 1, FIG. 3, and FIG. 4, the non-elastic area P1 is formed so that the first leg-surrounding elastic member 13 has a triangular portion without the tensile force at its center, and has the portion with the tensile force divided at the center. Still, no non-elastic area is formed in a substantially linear portion extending upward toward the upper edge 7 from the lower portions of the left and right bonded portions 5a and 5b to the center. The left and the right portions with the tensile force of the first leg-surrounding elastic member 13 as a result of the dividing are pulled toward each other by the tensile force including: tensile force in a substantially linear upward direction toward the upper edge 7 from the lower portion of the left bonded portion 5a to the center; and tensile force in a substantially linear upward direction toward the upper edge 7 from the lower portion of the right bonded portion 5b to the center. Thus, force of pulling down the center portion of the ventral member 2 is generated. As a result, as illustrated in FIG. 1, in the disposable diaper 1, the upper edge 7 of the ventral member 2 is positioned lower than the upper edge 8 of the dorsal member 3 with the center portion 7a being at the lowermost position.

As illustrated in FIG. 2 to FIG. 4, the non-elastic area P2 is formed to have a substantially rectangular shape with one longitudinal side disposed on the center line 11c of the center portion 3c of the dorsal member 3. The non-elastic area P2 has a left-right width that is the same as the width of the bottom line of the non-elastic area P1 having the triangular shape. With the non-elastic area P2 formed in the manner described above, the tensile force is lost in the center portion of the hip-surrounding elastic member 12c and in a part of the second leg-surrounding elastic member 14.

As illustrated in FIG. 2 to FIG. 4, the non-elastic area P3 is formed at the center of the dorsal member 3 in the waist-around direction, and has a shape of what is known as a home plate. The shape of a home plate is a combination of a substantially triangular shape with the apex on the side of the upper edge 8 and a substantially rectangular shape with the same width as the bottom line of the triangular shape. The apex of the triangular portion of the non-elastic area P3 is on the center line of the dorsal member 3 in the waist-around direction, and is positioned in an upper portion in the area including the hip-surrounding elastic member 12e. The bottom line of the triangular portion of the non-elastic area P3 is positioned between the hip-surrounding elastic member 12e and the hip-surrounding elastic member 12f. The bottom line of the rectangular portion of the non-elastic area P3 on a side of the lower edge 11c is positioned closer to the lower edge 11 than the hip-surrounding elastic member 12f. The non-elastic area P3 has a left-right width larger than those of the non-elastic areas P1 and P2. With the non-elastic area P3 formed in the manner described above, the hip-surrounding elastic member 12e is divided at the center portion in such a manner that the portion without the tensile force becomes longer toward the lower edge 11. The hip-surrounding elastic member 12f has the portion without the tensile force expanding in its center portion. The shape of the non-elastic area P3 is not limited to the shape of a home plate. The shape may be any tapered shape with the apex on the side of the upper edge 8 of the dorsal member 3.

As illustrated in FIG. 3 and FIG. 4, the hip-surrounding elastic member 12d of the dorsal member 3 has no non-elastic area formed, and thus has the longest portion with the tensile force. The hip-surrounding elastic member 12e is divided into left and right parts by the non-elastic area P3, in such a manner that the portion with the tensile force becomes shorter toward the lower edge 11. The hip-surrounding elastic member 12f is divided into left and right parts by the non-elastic area P3, and the length of the portion with the tensile force is the same as the portion with the tensile force closest to the lower edge 11 in the hip-surrounding elastic member 12e. With the non-elastic area P3 formed in the manner described above, in the dorsal member 3, the portion with the tensile force in the hip-surrounding elastic member 12 becomes shorter from the waist opening 6 toward the leg openings 9a and 9b on the lower side. Thus, as illustrated in FIG. 2, the dorsal member 3 has a shape gently widening from the waist opening 6 toward the leg openings 9a and 9b on the lower side, due to the contraction of the waist opening 6. Thus, the disposable diaper 1 can achieve excellent fitting for the wearer with a general body shape widening from the waist toward the upper ends of both legs.

As illustrated in FIG. 4, the non-elastic areas P1, P2, and P3 are formed at positions on the inner side of an absorbing body 20 disposed in the crotch member 4, when the crotch member 4 is bonded to the ventral member 2 and the dorsal member 3. With the non-elastic areas P1, P2, and P3 thus formed, the tensile force is lost in the major portion of the elastic member in the area in which the absorbing body 20 is disposed. Thus, the absorbing body 20 is prevented from deforming or contracting due to the tensile force of the elastic member.

As illustrated in FIG. 1 and FIG. 2, the waist-surrounding gathers 15 and 15 are formed in areas each being at and around the waist opening 6 of the ventral member 2 and the dorsal member 3 and including the hip-surrounding elastic member 12a or 12d. With the waist-surrounding gathers 15 and 15, the waist opening 6 of the disposable diaper 1 fits the wearer with an appropriate tightness. Thus, the diaper 1 is prevented from slipping down, and excretion or the like can be prevented from leaking through the waist opening 6.

As illustrated in FIG. 1 and FIG. 2, the hip-surrounding gathers 16 and 16 are each formed below the areas of the waist-surrounding gathers 15 and 15 of the ventral member 2 and the dorsal member 3, and formed in the area in which the hip-surrounding elastic members 12b and 12c or the hip-surrounding elastic members 12e and 12f are disposed. With the Hip-surrounding gathers 16 and 16, the ventral member 2 and the dorsal member 3 of the disposable diaper 1 fit the wearer with an appropriate tightness, and the excellent fitting of the diaper is achieved and the excretion or the like can be prevented from leaking, while preventing the crotch member 4 and the absorbing body 20 from deforming with the effects of the non-elastic areas P1, P2, and P3.

As illustrated in FIG. 1, the leg-surrounding gathers 17 and 17 are formed in areas being at and around the left and the right leg openings 9a and 9b of the ventral member 2 and including the first leg-surrounding elastic member 13. As illustrated in FIG. 1 and FIG. 2, the leg-surrounding gathers 18 and 18 are formed in areas being at and around the left and the right leg openings 9a and 9b of the dorsal member 3 and including the second leg-surrounding elastic member 14. With the leg-surrounding gathers 17 and 17 and 18 and 18, the left and the right leg openings 9a and 9b of the disposable diaper 1 fit the wearer with an appropriate tightness, and the excretion or the like can be prevented from leaking through the leg openings 9a and 9b.

As illustrated in FIG. 4 to FIG. 6, the crotch member 4 includes: a crotch sheet 4 as a crotch member main body; the absorbing body 20 obtained by wrappingan absorbing material 21 with an upper layer tissue 22a and a lower layer tissue 22b; a top sheet 23; a back sheet 24; a crotch elastic member 25 that is appropriately disposed in the crotch sheet 4 while being in the stretched state; and a ventral bonded portion J1 and a dorsal bonded portion J2.

As illustrated in FIG. 4, the crotch sheet 4 as the main body of the crotch member 4 is bonded onto the inner sheets 101 while bridging between the center portion 2c of the ventral member 2 and the center portion 3c of the dorsal member 3. The crotch sheet 4 has the ventral bonded portion J1 and the dorsal bonded portion J2 respectively bonded to the ventral member 2 and the dorsal member 3 with adhesive. The ventral bonded portion J1 is substantially the entire surface overlapping the ventral member 2. The dorsal bonded portion J2 is substantially the entire surface overlapping the dorsal member 3. The material of the crotch sheet 4 is not limited to the nonwoven fabric, and may be that used for the inner sheets 101 and the outer sheets 102 of the ventral member 2 and the dorsal member 3.

As illustrated in FIG. 4 to FIG. 6, the crotch sheet 4 has a substantially rectangular shape with a longitudinal direction extending in a direction in which the crotch sheet 4 bridges between the ventral member 2 and the dorsal member 3. The crotch sheet 4 has a ventral edge 4c positioned between the hip-surrounding elastic member 12a and the hip-surrounding elastic member 12b of the ventral member 2. The crotch sheet 4 has a dorsal edge 4d positioned in an upper portion in the area of the hip-surrounding elastic member 12e in the dorsal member 3. The crotch sheet 4 has both end portions in the width direction folded toward the absorbing body 20, and the resultant fold lines define the left and the right side edges 4a and 4b of the crotch member 4. Edges 4e and 4f of the folded pieces of the crotch sheet 4 are positioned at left and right outer edges of a largest width portion of the absorbing body 20 disposed on the crotch sheet 4. However, this should not be construed in a limiting sense, and the edges 4e and 4f of the folded pieces of the crotch sheet 4 may be positioned at inner edges of the absorbing body 20. The width of the crotch member 4, that is, the distance between the left and the right side edges 4a and 4b is larger than the largest width of the absorbing body 20. The shape of the crotch sheet 4 is not limited to the substantially rectangular shape, and may be a strip shape with a curved ventral edge or dorsal edge. The position and the size of the bonded portion between the crotch member 4 and the ventral member 2 or the dorsal member 3 are not limited to those described above, and may be changed as appropriate in accordance with the size and the shape of the diaper.

As illustrated in FIG. 4 to FIG. 6, the absorbing body 20 is disposed at the center of the crotch sheet 4. The absorbing body 20 has a longitudinal length that is slightly shorter than the longitudinal length of the crotch sheet 4. The length of the absorbing body 20 is preferably a length from the upper end of the groin to the upper end of sacrum of the wearer. The absorbing body 20 preferably has the largest width on the ventral side matching the width of the groin of the wearer. The absorbing body 20 has the largest dorsal width that is the same as the largest ventral width. However, this should not be construed in a limiting sense, and the largest dorsal width may be larger than the largest ventral width. The absorbing body 20 has a shape of an hourglass with a narrow portion 20b having the smallest width provided between ventral and dorsal wide portions 20a and 20c having the largest width. The absorbing body 20 is formed as a combination of absorbent paper, cotton pulp, a polymer absorbing material, and the like, and quickly absorbs and holds therein liquid bodies such as urine and blood.

As illustrated in FIG. 6, the absorbing body 20 includes the lower layer tissue 22b, the absorbing material 21, and the upper layer tissue 22a that are stacked in this order from the lower side, and has a structure in which folded pieces of the lower layer tissue 22b are bonded on the upper layer tissue 22a in an overlapping manner. The absorbing material 21 is formed by mixing crushed pulp with the polymer absorbing material. The upper layer tissue 22a is an absorbent paper covering the entire upper surface of the absorbing material 21. The lower layer tissue 22b is an absorbent paper covering the entire lower surface of the absorbing material 21.

As illustrated in FIG. 6, the crotch member 4 includes the crotch sheet 4, the back sheet 24, the absorbing body 20, and the top sheet 23 that are stacked in this order from the lower side, and has a structure in which the folded pieces of the crotch sheet 4 are bonded on the top sheet 23 in an overlapping manner. The top sheet 23 is a sheet member that covers the entire upper surface of the absorbing body 20, and is disposed on an inner side of the disposable diaper 1 to be in contact with the skin of the wearer. For example, the top sheet 23 is made of a liquid permeable material such as nonwoven fabric. Thus, urine, blood, and the like discharged into the diaper 1 transmit through the top sheet 23 to be sent to the absorbing body 20. The back sheet 24 is a sheet member that covers the entire lower surface of the absorbing body 20, and is fixed on the crotch sheet 4. For example, the back sheet 24 is made of a liquid impermeable material such as a polyethylene film. Thus, the liquid body that has transmitted through the top sheet 23 and failed to be absorbed by the absorbing body 20 is prevented from leaking to the crotch sheet 4 or to the outside.

As illustrated in FIG. 4 to FIG. 6, the crotch elastic member 25 includes crotch side edge elastic members 25a, ventral connection elastic members 25b, and crotch bottom elastic members 25c. All the crotch elastic members 25 are disposed while being stretched in the longitudinal direction of the crotch sheet 4. A dotted line portion in each crotch elastic member 25 represents a portion of each crotch elastic member 25 in the stretched state bonded to the crotch sheet 4 with adhesive and the like, and thus is a portion with the tensile force. The crotch elastic member 25 has higher tensile force than the hip-surrounding elastic member 12, the first leg-surrounding elastic member 13, and the second leg-surrounding elastic member 14. The ventral connection elastic member 25b has the highest tensile force in the crotch elastic member 25.

As illustrated in FIG. 4 to FIG. 6, the crotch side edge elastic members 25a are bonded between the crotch sheet 4 and left and right folded pieces of the crotch sheet 4. Each of the fold lines as the left and the right end portions 4a and 4b of the crotch member 4 and portions closer to the center is provided with a single crotch side edge elastic member 25a. The crotch side edge elastic member 25a has a portion with the tensile force that is separated from the ventral bonded portion J1 and the dorsal bonded portion J2. The ventral bonded portion J1 is bonded on the ventral member 2 in an overlapping manner. The dorsal bonded portion J2 is bonded on the dorsal member 3 in an overlapping manner. Thus, the crotch side edge elastic member 25a is not connected to the ventral bonded portion J1 and the ventral member 2, and is not connected to the dorsal bonded portion J2 and the dorsal member 3. Preferably, when the disposable diaper 1 is worn, the portion with the tensile force in the crotch side edge elastic member 25a has a ventral end portion positioned around the pubic bone of the wearer, and has a dorsal end portion positioned around the ischial bone.

As illustrated in FIG. 4 to FIG. 6, the ventral connection elastic members 25b are bonded between the crotch sheet 4 and the back sheet 24 with adhesive and the like. Portions closer to the center side than the left and right crotch side edge elastic members 25a are each provided with a single ventral connection elastic member 25b. The ventral connection elastic members 25b extend in the longitudinal direction of the crotch member 4 along the left and right outer edges of the largest width ventral portion 20a of the absorbing body 20. The portion with the tensile force of the ventral connection elastic member 25b has a ventral end portion at a position to be overlapped with the ventral bonded portion J1 of the crotch member 4. The ventral connection elastic member 25b is connected to the ventral member 2 with the crotch member 4 bonded to the ventral member 2 at the ventral bonded portion J1. The dorsal end portion of the ventral connection elastic member 25b does not overlap with the dorsal bonded portion J2 of the crotch member 4 and is not connected to the dorsal member 3. The dorsal end portion of the ventral connection elastic member 25b is positioned closer to the ventral bonded portion J1 than the dorsal end portion of the crotch side edge elastic member 25a. Preferably, when the disposable diaper 1 is worn, the portion with the tensile force in the ventral connection elastic member 25b has the ventral end portion positioned in the upper portion of the groin of the wearer, and has the dorsal end portion positioned around the ischial bone.

As illustrated in FIG. 1, the tensile force of the ventral connection elastic members 25b connected to the ventral member 2 acts in a direction crossing the hip-surrounding elastic member 12 and the first leg-surrounding elastic member 13 of the ventral member 2. Thus, the ventral member 2 is pulled toward the crotch member 4, whereby the center portion 7a of the upper edge 7 of the ventral member 2 is pulled down.

When the disposable diaper 1 is worn by the wearer, the pair of ventral connection elastic members 25b and 25b of the crotch member 4 are connected to the ventral member 2, and the ventral member 2 and the crotch member 4 are pulled toward each other. Thus, excellent fitting can be achieved for the wearer. The pair of ventral connection elastic members 25b and 25b are positioned outside of both edges of the largest width ventral portion of the absorbing body 20, and are positioned outside of both sides of the groin of the wearer. The crotch member 4 has the left and the right side portions with the crotch side edge elastic members 25a and the ventral connection elastic members 25b bonded along the longitudinal direction, whereby gathers are formed. The left and the right side portions of the crotch member 4 where the gathers are formed stand like both side walls due to the tensile force of the ventral connection elastic member 25b connected to the ventral member 2, and thus functions as standing cuff. The crotch side edge elastic member 25a is not connected to the ventral member 2 or to the dorsal member 3. Thus, the width of the crotch member 4 can be increased/decreased in accordance with the body shape and the movement of the wearer. Thus, the crotch member 4 has a standing form to cover side and bottom surfaces of a portion from the ventral side to the crotch of the wearer, whereby excellent fitting can be achieved. The standing form of the crotch member 4 is maintained by the tensile force of the crotch side edge elastic member 25a and the ventral connection elastic member 25b. Thus, the excretion can be prevented from leaking through the left and the right side edges 4a and 4b of the crotch member 4.

As illustrated in FIG. 4, the portion of the crotch member 4 bonded to the dorsal member 3 has a larger width than the largest dorsal width of the absorbing body 20. In the present embodiment, the absorbing body 20 has the largest dorsal width that is the same as the largest ventral width. However, this should not be construed in a limiting sense, and the largest dorsal width may be larger than the largest ventral width. The crotch elastic member 25 is not connected to the dorsal member 3. Thus, the portion of the crotch member 4 connected to the dorsal member 3 is not moved by the contraction of the crotch elastic member 25 due to the movement of the wearer. Thus, the disposable diaper 1 can cover the buttocks of the wearer with the large dorsal width of the crotch member 4.

As illustrated in FIG. 4 to FIG. 6, the crotch bottom elastic members 25c are bonded between the crotch sheet 4 and the back sheet 24. Portions closer to the center than the ventral connection elastic members 25b and 25b are each provided with a single crotch bottom elastic member 25c. A portion with the tensile force in the crotch bottom elastic member 25c is preferably disposed in accordance with the shape of the absorbing body disposed on the crotch member 4. When the absorbing body 20 has the shape of an hourglass, the crotch bottom elastic members 25c are disposed in gap areas between the left and the right side edges of the narrow portion 20b as the smallest width portion of the absorbing body 20 and the ventral connection elastic members 25b positioned outside of the edges of the largest width ventral portion of the absorbing body 20, without overlapping with the absorbing body 20. With the crotch bottom elastic members 25c, gathers are formed in the gap areas, whereby the bottom portion of the crotch member 4 is prevented from loosening and is fit to the wearer, and a static shape of the bottom portion of the crotch member 4 is achieved. The number of crotch bottom elastic members 25c formed on each of the left and right sides is not limited to one and may be more than one. The crotch bottom elastic member 25c may be omitted when the difference between the largest ventral width and the smallest ventral width of the absorbing body 20 is small and thus the gap area is small.

In the present embodiment, two crotch side edge elastic members 25a, one ventral connection elastic member 25b, and one crotch bottom elastic member 25c are formed on each of left and right sides. However, this should not be construed in a limiting sense.

The crotch elastic member 25 is made of a flexible thread-like material such as natural rubber, polyurethane resin, and flexible hot melt, as in the case of the hip-surrounding elastic member 12, the first leg-surrounding elastic member 13, and the second leg-surrounding elastic member 14. The elastic member is not limited to the thread-like form, and an elastic member in a strip form or a sheet form may be used. The entire area where the crotch gathers 19 are formed may be made of a material such as a flexible sheet. When the elastic member in a sheet form is used or when the entire area where the crotch gathers 19 are formed is made of a material such as a flexible sheet, the tensile force acting in an extending direction of each crotch elastic member 25 in a thread form is required.

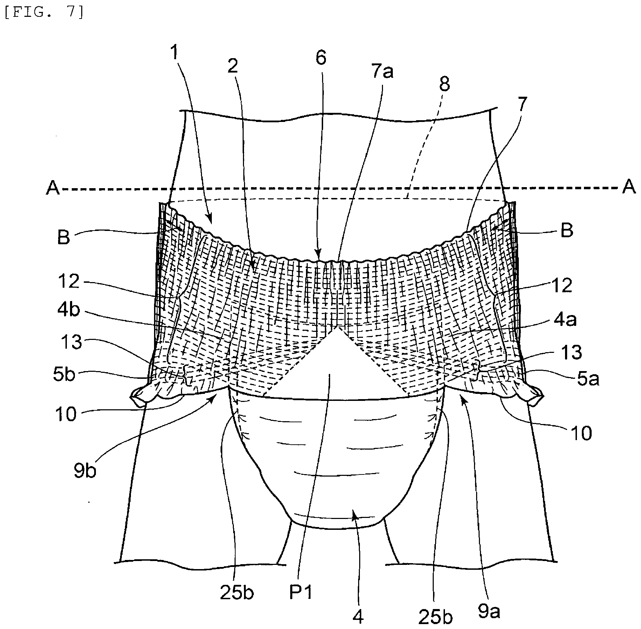

FIG. 7, FIG. 8, and FIG. 9 are respectively a front view, a back view, and a left side view illustrating a state where the disposable diaper 1 according to the present embodiment is worn. The dashed line A-A represents a horizontal line around the waist of the wearer. The dashed lines B are lines extending from the iliac crest along anterior superior iliac spines of the wearer. The dashed line Z-Z is a center line in a vertical direction of the wearer.

As illustrated in FIG. 1 and FIG. 7, in the disposable diaper 1 according to the present embodiment, the first leg-surrounding elastic member 13 of the ventral member 2 extends substantially linearly toward the upper edge 7 from both bonded portions 5a and 5b to the center in the waist-around direction. The first leg-surrounding elastic member 13 is longer than the hip-surrounding elastic member 12. The first leg-surrounding elastic member 13 is divided at the center by the non-elastic area P1, and the portions extending substantially linearly toward the upper edge 7 from the bonded portions 5a and 5b to the center in the waist-around direction are pulled to each other. Thus, the center portion 7a of the upper edge 7 of the ventral member 2 is pulled down. The ventral connection elastic members 25b of the crotch member 4 are connected to the ventral member 2, and pull down the center portion 7a of the upper edge 7 of the ventral member 2. On the other hand, the tensile force in the waist-around direction acts on the ventral member 2 due to the hip-surrounding elastic member 12. Thus, the center portion 7a of the upper edge 7 of the ventral member 2 is prevented from being pulled down. In the non-elastic area P1, a portion with the tensile force of the hip-surrounding elastic member 12 becomes shorter and thus the tensile force of the hip-surrounding elastic member 12 becomes smaller from the waist opening 6 toward the leg openings 9a and 9b on the lower side. This contributes to the pulling down of the center portion 7a of the upper edge 7 of the ventral member 2. The hip-surrounding elastic member 12 of the ventral member 2 has higher tensile force than the hip-surrounding elastic member 13 of the dorsal member 3. Thus, the ventral member 2 contracts more in the waist-around direction than the dorsal member 3. Thus, the upper portions of the bonded portions 5a and 5b extending along both end portions of the disposable diaper 1 in the waist-around direction are pulled toward the center of the ventral member 2. This contributes to the pulling down of the center portion 7a of the upper edge 7 of the ventral member 2. The upper edge 7 of the ventral member 2 is pulled down to and stays at a position where the effects of the first leg-surrounding elastic member 13, the ventral connection elastic member 25b, the hip-surrounding elastic member 12, and the non-elastic area P1 are balanced. Thus, as illustrated in FIG. 1 and FIG. 7, the disposable diaper 1 has the upper edge 7 of the ventral member 2 positioned lower than the upper edge 8 of the dorsal member 3 with the center portion 7a at the lowermost portion. In the present embodiment, the state of the disposable diaper 1 where the center portion 7a of the upper edge 7 of the ventral member 2 is positioned lower than the upper edge 8 of the dorsal member 3 is achieved by using all of the first leg-surrounding elastic member 13, the ventral connection elastic member 25b, the hip-surrounding elastic member 12, and the non-elastic area P1. However, this should not be construed in a limiting sense. The state may be achieved by using the first leg-surrounding elastic member 13, the ventral connection elastic member 25b, the hip-surrounding elastic member 12, and the non-elastic area P1, individually or in combination.

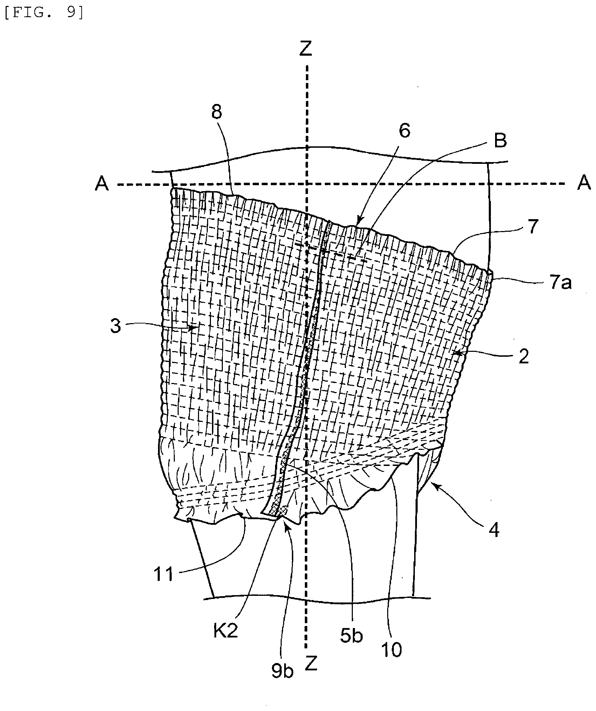

As illustrated in FIG. 7 and FIG. 9, when the disposable diaper 1 is worn, the upper edge 7 of the ventral member 2 is positioned lower than the line A around the waist of the wearer. The upper edge 7 of the ventral member 2 extends downward from both bonded portions 5a and 5b in accordance with an angle of the lines B extending from the iliac crest through the anterior superior iliac spines of the wearer in such a manner that the center portion 7a of the upper edge 7 is positioned at the lowermost portion. Thus, in the disposable diaper 1, the hip-surrounding elastic member 12 of the ventral member 2 does not pass through the waist, and thus does not compress the waist. The waist opening 6 hangs on and stays at the iliac crest, and thus the stable wearing position of the disposable diaper 1 is achieved. The leg-surrounding elastic member 13 is likely to return to the original position upon being in contact with the moved leg of the wearer, and thus the stable wearing position of the disposable diaper 1 is achieved. The disposable diaper 1 has a short rise length especially on the ventral side, and thus does not stick out from pants and skirts. The disposable diaper 1 features a small area covering the ventral side of the wearer, and thus can achieve excellent fitting and can be manufactured with a small amount of resource.

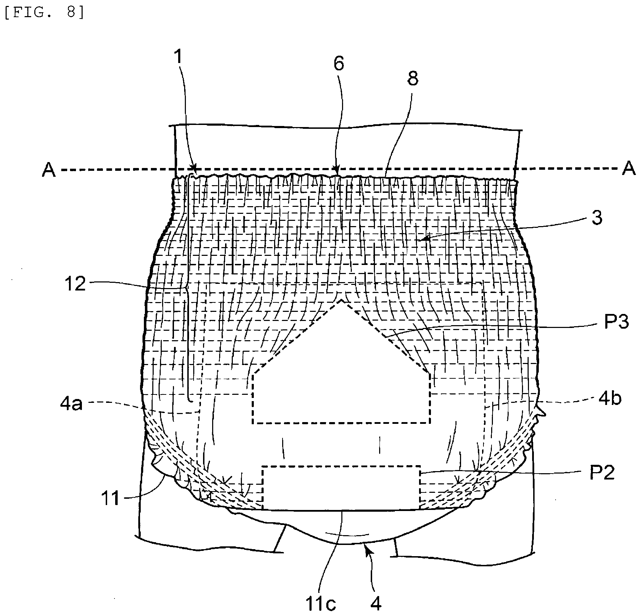

As illustrated in FIG. 1 and FIG. 2, the non-elastic area P1 has a substantially triangular shape with the apex on the side of the upper edge 7 of the ventral member 2. The non-elastic area P3 has a shape of a substantially home plate tapered toward the upper edge 8 of the dorsal member 3. In each of the areas, the portion with the tensile force in the hip-surrounding elastic member 12 becomes smaller, and thus the tensile force of the hip-surrounding elastic member 12 in the waist-around direction becomes weaker from the waist opening 6 toward the leg openings 9a and 9b on the lower side. Thus, the ventral member 2 and the dorsal member 3 each has a shape widening from the waist opening 6 toward the leg openings 9a and 9b on the lower side with the waist opening 6 contracted. Thus, as illustrated in FIG. 7 and FIG. 8, the disposable diaper 1 can be well fit to the body shape of the wearer widening from the waist toward the upper ends of both legs. Furthermore, the waist opening 6 can be prevented from being pulled down when the wearer sits down or bends over. As illustrated in the back view in FIG. 8, when the wearer wears the disposable diaper 1, the dorsal member 3 has an enough area to cover the pelvis of the wearer, with the upper edge 8 substantially horizontally positioned slightly below the horizontal line A-A on the waist of the wearer.

The waist opening 6 is defined by the upper edge 7 of the ventral member 2 and the upper edge 8 of the dorsal member 3. As illustrated in FIG. 9, when the disposable diaper 1 is worn, the waist opening 6 has a line inclined by the center portion 7a of the upper edge 7 of the ventral member 2 lowered in accordance with an angle of the lines B, extending from the iliac crest along the anterior superior iliac spines, with respect to the horizontal line A-A around the waist of the wearer. The hip-surrounding elastic member 12 in the ventral member 2 has higher tensile force than the tensile force of the hip-surrounding elastic member 12 in the dorsal member 3. Thus, the bonded portion 5b between the ventral member 2 and the dorsal member 3 extends in a line inclined with respect to the center line Z-Z in the vertical direction of the wearer, with the upper end portion disposed on the ventral side and the lower end portion disposed on the dorsal side. The upper and lower length, that is, the rise length is small in a portion around the boundary between the ventral member 2 and the crotch member 4, and is large at the center portion of the dorsal member 3. Thus, the leg opening 9b defined by the lower edge 10 of the ventral member 2 and the lower edge 11 of the dorsal member 3 extends in an inclined line. In this manner, the disposable diaper 1 being worn is inclined with respect to the center line Z-Z of the wearer. All things considered, the rise length is smaller in the ventral member 2 and is larger in the dorsal member 3, without being too small or too large.

As described above, in the present embodiment, the disposable diaper 1 with a small rise length that does not excessively compress the waist of the wearer, achieves a stable wearing position, and can be manufactured with a small amount of resource can be provided.

Next, a method for manufacturing a disposable undergarment according to one embodiment of the present invention is described.

As illustrated in FIG. 3, each of the elastic members 12, 13, and 14 is disposed and bonded on the inner sheet 101 or the outer sheet 102. In this process, adhesive and the like may be applied on the surface of each elastic member in a thread-like form, or may be applied on surfaces of the inner sheet 101 and the outer sheet 102 facing the elastic members by spray coating and the like. Each elastic member in a thread-like form is bonded to the inner sheet 101 and the outer sheet 102 in a state of being stretched in accordance with the required tensile force. Next, a layered web is formed with the inner sheets 101 and the outer sheets 102 attached to each other with the elastic members 12, 13, and 14 provided in between. Next, the layered web is processed so that the elastic member 12, 13, or 14 partially loses the tensile force, whereby the non-elastic areas P1, P2, and P3 are formed. For example, processes including providing multiple protrusions, cutting the elastic member with a cutter blade or the like, heat sealing, and the like may be appropriately selected for a process with which the elastic member loses the tensile force. The layered web is formed with a layered body of a single pitch, illustrated in FIG. 3, arranged side by side in a flow direction corresponding to the left and right direction in FIG. 3. The layered web is cut into individual pitches at planned cutting lines at positions of the end portions 2a and 3a and the end portions 2b and 3b illustrated in FIG. 3. Then, the layered web is cut along the planned cutting line C to be divided into a half layered web including the ventral members 2 arranged in series and a half layered web including the dorsal members 3 arranged in series.

Next, as illustrated in FIG. 4, the distance between the half layered web including the ventral members 2 and the half layered web including the dorsal members 3 is widened. Then, the crotch member 4 has one end portion bonded to the half layered web including the ventral members 2 arranged in series and has the other end portion bonded to the half layered web including the dorsal members 3 arranged in series with adhesive such as hot melt, with a gap provided in each pitch in the flow direction. One pitch corresponds to a distance between both end portions 2a and 2b and both end portions 3a and 3b of the disposable diaper 1 in the waist-around direction or the left and right direction. Thus, a continuous body is formed that includes the disposable diapers 1, opened as illustrated in FIG. 4, disposed in series in the flow direction as the left and right direction in FIG. 4. FIG. 4 illustrates portions to be the bonded portions 5a and 5b where the ventral member 2 and the dorsal member 3 are bonded in an overlapping manner. The ventral member 2 and the dorsal member 3 are partially bonded together with the positioning bonded portions K1 and K2 before the bonded portions 5a and 5b are formed. Portions to be the positioning bonded portions K1 and K2 are illustrated to be at the end portions of the lower edge 10 and the lower edge 11 that face each other, in such a manner as to partially overlap portions to be the bonded portions 5a and 5b.

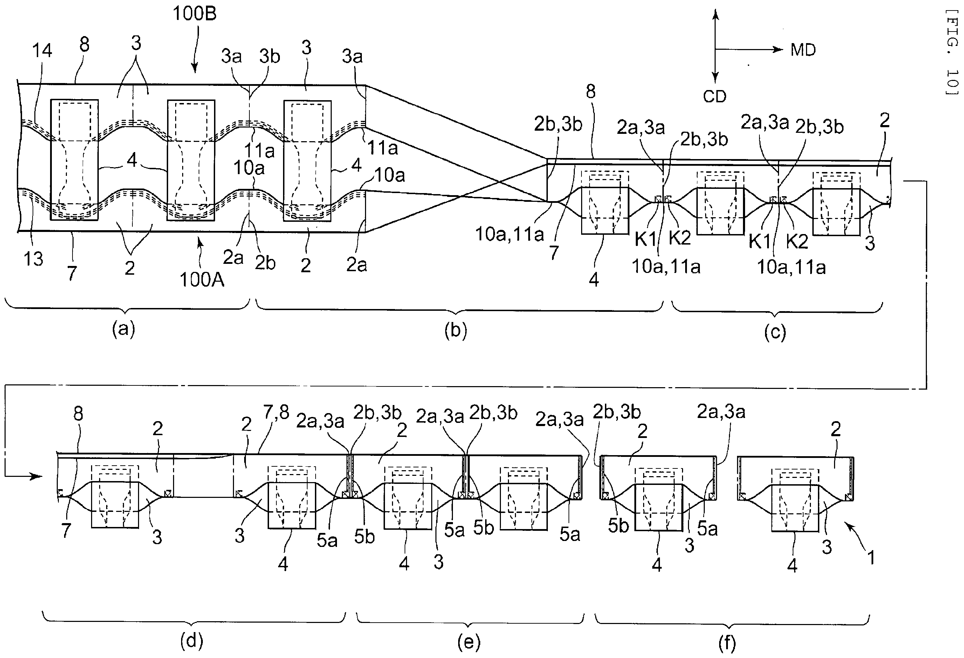

FIG. 10 is a diagram illustrating manufacturing steps for the disposable undergarment according to the present embodiment. In FIG. 10, an arrow MD represents a machine direction as a direction in which the web flows. An arrow CD in a direction orthogonal to the arrow MD represents a width direction of the web. The length of one pitch in the flow direction corresponds to the length of each of the upper edges 7 and 8 of the ventral member 2 and the dorsal member 3 of a single disposable diaper 1. In FIG. 10, the elastic members, the non-elastic areas, and the like are omitted as appropriate.

In step (a) in FIG. 10, the half layered web 100A and the half layered web 100B are arranged in parallel with each other. The half layered web 100A includes the ventral members 2 arranged in series in the flow direction. The half layered web 100B includes the dorsal members 3 arranged in series in the flow direction. The crotch members 4 that are in the same orientation and bonded at the same bonding positions are each provided in each pitch in the flow direction.

The half layered web 100A has protruding portions protruding toward the half layered web 100B on the opposite side, and the protruding portions are each formed between adjacent ones of portions where the crotch members 4 are bonded. In the protruding portion, the first leg-surrounding elastic member 13 extends in such a manner as to be meandered with respect to the flow direction. The half layered web 100B has recessed portions each formed between adjacent ones of portions where the crotch members 4 are bonded. In the recessed portion, the second leg-surrounding elastic member 14 extends in such a manner as to be meandered with respect to the flow direction.

The present inventors have found the following problem that occurs when the crotch members 4 are folded in two in a direction orthogonal to the flow direction so that the half layered web 100A including the ventral members 2 arranged in series is overlapped with the half layered web 100B including the dorsal members 3 arranged in series. Specifically, in the protruding portions of the half layered web 100A, the tensile force does not appropriately act in the flow direction (direction MD) for conveying the web and in the width direction (direction CD). Furthermore, in the protruding portion, the tensile force of the first leg-surrounding elastic member 13 meandering with respect to the flow direction acts to make the portion kinked or wrinkled. Thus, when the upper edge 7 of the half layered web 100A is aligned with the upper edge 8 of the half layered web 100B, both end lines 10a of the lower edge of the half layered web 100A do not match both end lines 11a of the lower edge of the half layered web 100B. As a result, problems such as misalignment, wrinkling, and even a failure to appropriately form the bonded portions occur.

To solve the problems, the manufacturing method according to the present embodiment includes step (b) illustrated in FIG. 10, in which the half layered web 100A is overlapped on the half layered web 100B with the lower edges 10a and 10a of both end portions 2a and 2b of the ventral member 2 of the half layered web 100A positioned to be aligned with the lower edges 11a and 11a of both end portions 3a and 3b of the dorsal member 3 of the half layered web 100B in each pitch in the flow direction. At this point, the upper edge 7 of both end portions 2a and 2b of the ventral member 2 is not aligned with the upper edge 8 of both end portions 3a and 3b of the dorsal member 3.

Next, in step (c) in FIG. 10, the portions where the lower edges 10a and 10a of both end portions 2a and 2b of the ventral member 2 of the half layered web 100A are aligned with the lower edges 11a and 11a of both end portions 3a and 3b of the dorsal member 3 of the half layered web 100B are partially bonded, in each pitch in the flow direction. For example, the partial bonding is performed as follows. Specifically, heat sealing is performed at each of positions that are at and around the lower edges 10a and 11a of the ventral member 2 and the dorsal member 3 of the half layered webs 100A and 100B overlapped with each other and are on both end portions 2a and 3a and 2b and 3b adjacent to each other, in each pitch in the flow direction. Thus, in each pitch in the flow direction, the positioning bonded portions K1 and K2 are formed as bonded portions that are at and around both end portions 2a and 2b and 3a and 3b of the ventral members 2 in the half layered web 100A and the dorsal members 3 in the half layered web 100B in the flow direction, and around the opposite edges of the ventral member 2 and the dorsal member 3.

Next, in step (d) in FIG. 10, the upper edge 7 of the ventral member 2 of the half layered web 100A is stretched toward the upper edge 8 of the dorsal member 3 of the half layered web 100B, so that the upper edge 7 of the ventral member 2 is aligned with the upper edge 8 of the dorsal member 3.

Next, in step (e) in FIG. 10, the bonded portions 5a and 5b are formed through ultrasonic sealing, heat sealing, by using adhesive (hot melt), or the like along both end portions 2a and 3a and 2b and 3b of the ventral member 2 of the half layered web 100A and the dorsal member 3 of the half layered web 100B aligned to each other. With the bonded portions 5a and 5b, the ventral member 2 of the half layered web 100A and the dorsal member 3 of the half layered web 100B are bonded to each other in each pitch in the flow direction.

Next, in step (f) in FIG. 10, the ventral member 2 of the half layered web 100A and the dorsal member 3 of the half layered web 100B bonded to each other at the bonded portions 5a and 5b are cut at substantially the center of the bonded portions 5a and 5b in each pitch in the flow direction. Thus, the disposable diapers 1 are manufactured by being separated into individual pieces that are in the same orientation. The elastic members 12, 13, and 14 contract when the cutting is performed in each pitch in the flow direction. As a result, as illustrated in FIG. 4, the elastic members 12, 13, and 14 are not provided in areas between the bonded portion 5a and the end portion 2a, between the bonded portion 5a and the end portion 3a, between the bonded portion 5b and the end portion 2b, or between the bonded portion 5b and the end portion 3b. Alternatively, the elastic members 12, 13, and 14 may remain in these areas.

The positioning bonded portion according to the present invention is not limited to the positioning bonded portions K1 and K2 according to the present embodiment, and may be any portion with which the half layered webs 100A and 100B overlapped with each other can be positioned and the upper edge 7 of the ventral member 2 and the upper edge 8 of the dorsal member 3 can be aligned with each other when the upper edge 7 of the ventral member 2 of the half layered web 100A is stretched toward the upper edge 8 of the dorsal member 3 of the half layered web 100B. The position and the range where the positioning bonded portion is formed, and how the positioning bonded portion is bonded may be appropriately selected. For example, in each pitch in the flow direction, positioning bonded portions disposed on the adjacent both end portions 2a and 3a and 2b and 3b may be formed at and around the lower ends 10a and 11a of the ventral member 2 and the dorsal member 3 of the half layered webs 100A and 100B overlapped with each other. A plurality of small positioning bonded portions may be formed. The bonding method is not limited to the heat sealing, and may be ultrasonic sealing, hot melt adhesive, or the like.

As described above, with the manufacturing method for a disposable undergarment according to the present embodiment, the disposable diapers 1 that are less likely to have misalignment and wrinkling at the bonded portions 5a and 5b, can be manufactured in series in the same orientation.

### (Second embodiment)

Next, a disposable undergarment according to another embodiment of the present invention is described.

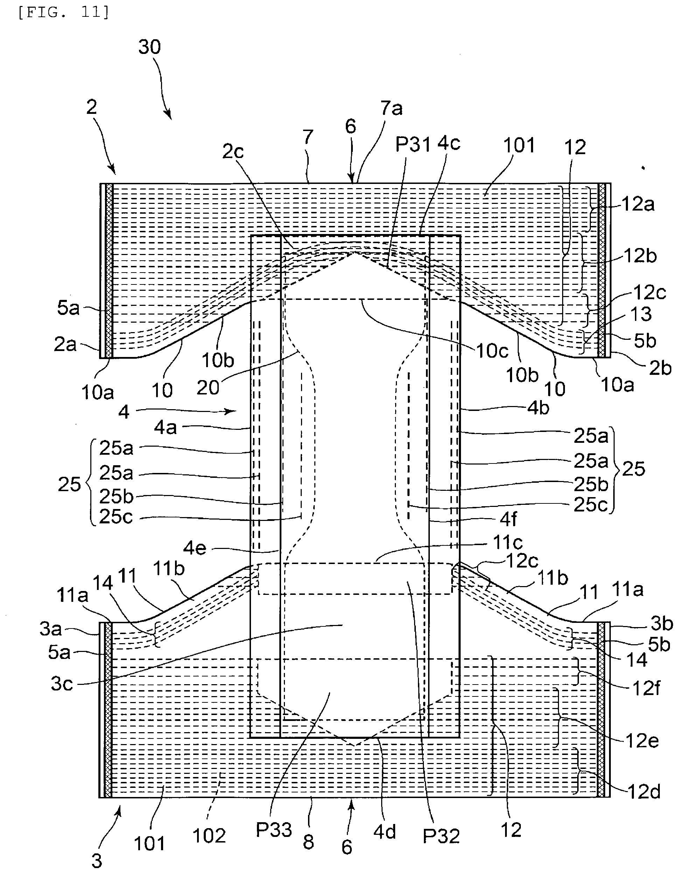

FIG. 11 is a diagram illustrating a disposable diaper 30 according to the present embodiment in an open state with elastic members in a stretched state. Components with configurations and functions that are the same as those in the embodiment described above are denoted with the same reference numerals, or the redundant description is omitted as appropriate. The disposable diaper 30 according to the present embodiment is the same as the disposable diaper 1 according to the embodiment described above, except that non-elastic areas P31, P32, and P33 with modified shapes are provided.

The disposable diaper 30 according to the present embodiment is different from the disposable diaper 1 according to the embodiment described above in that the non-elastic area P31 has a triangular shape with the apex positioned below the center of the first leg-surrounding elastic member 13, and an upper-center portion of each of the first leg-surrounding elastic member 13 and the hip-surrounding elastic member 12b is not divided. The non-elastic areas P31, P32, and P33 are wider than the largest width portion of the absorbing body 20 and extend to the vicinity of the left and the right edges 4a and 4b of the crotch member 4. The triangular portion of the non-elastic area P33 having a shape of a home plate is formed to have the apex disposed beyond the absorbing body 20 and the ventral edge 4d of the crotch member 4.

In the disposable diaper 30, the non-elastic area P31 does not divide the upper-center portion of the first leg-surrounding elastic member 13. Thus, the tensile force of the first leg-surrounding elastic member 13 that is not divided acts in such a manner that the first leg-surrounding elastic member 13 linearly extends from the lower portion of the left bonded portion 5a to the lower portion of the right bonded portion 5b. Thus, the force of pulling up the center portion of the ventral member 2 is stronger than that achieved with the non-elastic area P1 of the disposable diaper 1. Furthermore, the hip-surrounding elastic member 12b is also not divided by the non-elastic area P31. Thus, the center portion 7a of the upper edge 7 of the ventral member 2 can be positioned lower than the upper edge 8 of the dorsal member 3 in the disposable diaper 30 with the balance between the undivided members appropriately adjusted.

The disposable diaper 30 is different from the disposable diaper 1 in that the non-elastic areas P31, P32, and P33 are wider than the largest width portion of the absorbing body 20 to extend to the vicinity of the left and the right edges 4a and 4b of the crotch member 4. With the non-elastic area P31, the second leg-surrounding elastic member 14 extending in the area of the ventral member 2 that overlaps with the crotch member 4 entirely loses the tensile force. Thus, a connected portion between the ventral member 2 and the crotch member 4 can be prevented from wrinkling. With the non-elastic area P32, the tensile force can be lost in a large portion of the elastic member around the center line 11C of the dorsal member 3. Thus, the portion of the crotch member 4 connected to the dorsal member 3 can be prevented from contracting in the waist-around direction, and thus the buttocks of the wearer can be widely covered. With the non-elastic area P33, the hip-surrounding elastic members 12e and 12f extending in the portion of the crotch member 4 that overlaps with the dorsal member 3 can lose their effect across a large portion. Thus, the crotch member 4 and the absorbing body 20 can be prevented from contracting or deforming.

In the disposable diaper 30, the triangular portion of the non-elastic area P33 is formed to have the apex disposed beyond the absorbing body 20 and the ventral edge 4d of the crotch member 4. Thus, the portion without the tensile force in the hip-surrounding elastic members 12e and 12f of the dorsal member 3 has a large left-right width and a large upper and lower range. Thus, in the dorsal member 3, the tensile force of the hip-surrounding elastic member 12 weakens at a higher rate from the waist opening 6 toward the leg openings 9a and 9b. All things considered, the shape widening from the waist opening 6 toward the leg openings 9a and 9b with the waist opening 6 contracted can be more easily achieved.

In the disposable diaper 1 and in the disposable diaper 30, the center line 10c and the center line 11c in the areas of the ventral member 2 and the dorsal member 3 that overlap with the crotch member 4 linearly extend in parallel with the waist-around direction. However, this should not be construed in a limiting sense. The center line 10c of the ventral member 2 may be formed in a curved shape to gently protrude toward the side of the upper edge 7. The center line 11c of the dorsal member 3 may be formed in a curved shape to gently protrude toward the side opposite to the upper edge 8 of the dorsal member 3, to match the center line 10c. The disposable diaper 30 and the modifications described above can be manufactured through a method similar to that for the disposable diaper 1 described above.

### (Third embodiment)

Next, a disposable diaper according to still another embodiment of the present invention is described.

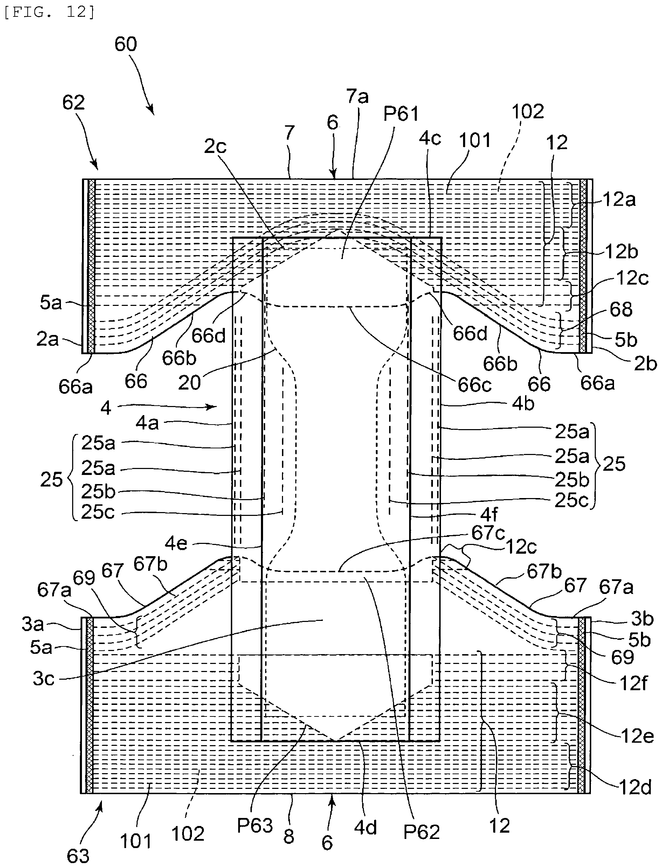

FIG. 12 is a diagram illustrating a disposable diaper 60 according to the present embodiment in an open state with an elastic member in a stretched state. Components with configurations and functions that are the same as those of the disposable diaper 1 according to the embodiment described above are denoted with the same reference numerals, or the redundant description is omitted as appropriate. The disposable diaper 60 according to the present embodiment is different from the disposable diaper 1 described above in the following points. Specifically, a lower edge 66 of a ventral member 62 and a lower edge 67 of a dorsal member 63 with modified shapes are provided. The number of components of the hip-surrounding elastic member 12 in the ventral member 62 is different. A first leg-surrounding elastic member 68 and a second leg-surrounding elastic member 69 with modified configurations are provided. Non-elastic areas P61, P62, and P63 with modified shapes are provided.

As illustrated in FIG. 12, the disposable diaper 60 according to the present embodiment includes the ventral member 62, the dorsal member 63, and the crotch member 4. The ventral member 62 and the dorsal member 63 are bonded to each other at both bonded portions 5a and 5b. The waist opening 6 is defined by the upper edge 7 of the ventral member 62 and the upper edge 8 of the dorsal member 63. The left and the right leg openings are each defined by the lower edge 66 of the ventral member 62 or the lower edge 67 of the dorsal member 63 and the left side edge 4a or the right side edge 4b of the crotch member 4. The hip-surrounding elastic member 12 extends in the ventral member 62 and the dorsal member 63. The first leg-surrounding elastic member 68 extends in the left and the right leg openings of the ventral member 62. The second leg-surrounding elastic member 69 extends in the left and the right leg openings of the dorsal member 63. The crotch member 4 includes the absorbing body 20, the crotch elastic member 25, and the like.

The disposable diaper 60 according to the present embodiment has the lower edge 66 of the ventral member 62 including: a pair of both end lines 66a and 66a substantially in parallel with each other; a pair of inclined lines 66b and 66b that gradually extend upward from both end lines 66a and 66a to the left and the right end portions 4a and 4b of the crotch member 4; a center line 66c that extends substantially horizontally; and curved lines 66d and 66d that extend downward from the inclined lines 66b and 66b toward the center line 66c while being gently curved, and thus is different from the lower edge 10 of the ventral member 2 of the disposable diaper 1. The disposable diaper 60 features the inclined lines 66b and 66b that are more sharply inclined than the counterparts in the disposable diaper 1. The rise length, that is, the distance between the upper edge 7 and the lower edge 66 at the intersecting point between the curved lines 66d and 66d and the left and the right end portions 4a and 4b of the crotch member 4 is smaller than that in the disposable diaper 1. Thus, the wearer wearing the disposable diaper 60 can easily move his or her legs.

The disposable diaper 60 includes the first leg-surrounding elastic member 68 extending upward from the bonded portions 5a and 5b of the ventral member 62 to a center-lower portion of the hip-surrounding elastic member 12a and to a portion above the crotch member 4. Thus, the first leg-surrounding elastic member 68 extends to a position closer to the upper edge 7 than the first leg-surrounding elastic member 13 of the disposable diaper 1. The first leg-surrounding elastic member 68 includes elastic members in a thread-like form that are arranged at an arrangement interval longer than that in the disposable diaper 1. The first leg-surrounding elastic member 68 includes the non-elastic area P61 only in a lower end portion thereof. Thus, the tensile force acting in such a manner that the first leg-surrounding elastic member 68 linearly extends from the lower portion of the left bonded portion 5a of the first leg-surrounding elastic member 68 to the lower portion of the right bonded portion 5b is stronger than those in the disposable diaper 1 and the disposable diaper 30 according to the embodiments described above. All things considered, the center portion 7a of the upper edge 7 of the ventral member 62 is pulled down with even higher force.

In the disposable diaper 60, the hip-surrounding elastic members 12b and 12c of the ventral member 62 each include one less elastic members in a thread-like form, compared with that in the disposable diaper 1. Thus, the tensile force of the hip-surrounding elastic member 12 of the ventral member 62 is weaker than that in the disposable diaper 1. Thus, the disposable diaper 60 can have the center portion 7a of the upper edge 7 of the ventral member 62 positioned more on the lower side of the upper edge 8 of the dorsal member 63 than in the disposable diaper 1 and in the disposable diaper 30. The disposable diaper 60 can be manufactured through a method similar to that for the disposable diaper 1 described above.

### (Fourth embodiment)

Next, a disposable undergarment according to yet still another embodiment of the present invention is described below.

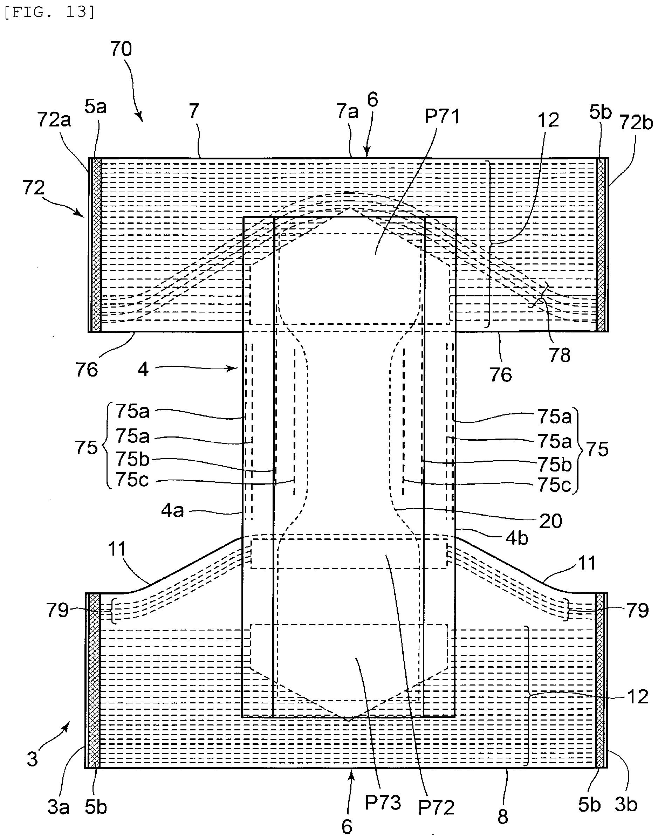

FIG. 13 is a diagram illustrating a disposable diaper 70 according to the present embodiment in an open state with an elastic member in a stretched state. Components with configurations and functions that are the same as those of the disposable diaper 1 according to the embodiment described above are denoted with the same reference numerals, or the redundant description is omitted as appropriate. In the disposable diaper 1 according to the embodiment described above, as illustrated in FIG. 4, the ventral member 2 in the stretched state has the rectangular shape with one longitudinal side recessed inward in a substantially trapezoidal form. As illustrated in FIG. 13, the disposable diaper 70 according to the present embodiment includes a ventral member 72 having a substantially rectangular shape.

As illustrated in FIG. 13, the disposable diaper 70 according to the present embodiment includes the ventral member 72, the dorsal member 3, and the crotch member 4. The ventral member 72 and the dorsal member 3 are bonded to each other at both bonded portions 5a and 5b. The waist opening 6 is defined by the upper edge 7 of the ventral member 72 and the upper edge 8 of the dorsal member 3. The left and the right leg openings are defined by the lower edge 76 of the ventral member 72 and the lower edge 11 of the dorsal member 3, and the left and the right side edges 4a and 4b of the crotch member 4. The hip-surrounding elastic member 12 extends in the ventral member 72 and the dorsal member 3. A second leg-surrounding elastic member 79 extends in the left and the right leg openings of the dorsal member 3. The crotch member 4 includes the absorbing body 20, a crotch elastic member 75, and the like.

A first elastic member 78 in the disposable diaper 70 does not extend in the left and the right leg openings of the ventral member 72, and extends upward toward the upper edge 7 to the center in the waist-around direction from the bonded portions 5a and 5b of the ventral member 72. The first elastic member 78 extends upward above the crotch member 4 of the ventral member 72, and is only partially divided by a non-elastic area P71 that has a shape of a home plate in a tapered form with the apex on the side of the upper edge 7. The tensile force of a portion of the first elastic member 78 that is not divided acts in such a manner that the first elastic member 78 linearly extends from the lower portion of the left bonded portion 5a to the lower portion of the right bonded portion 5b. Thus, in the disposable diaper 70, the center portion 7a of the upper edge 7 of the ventral member 72 is pulled down with larger force than that in the disposable diaper 1. Thus, the disposable diaper 70 can have the center portion 7a of the upper edge 7 of the ventral member 72 pulled even further downward with respect to the upper edge 8 of the dorsal member 3.

In the disposable diaper 70, non-elastic areas P71 to P73 are wider than the largest width of the absorbing body 20 and extend to the vicinity of the left and the right edges 4a and 4b of the crotch member 4. The non-elastic area P71 has a shape of a home plate in a tapered form with the apex on the side of the upper edge 7 of the ventral member 72. The non-elastic area P72 has a rectangular shape. The non-elastic area P73 has a shape of a home plate in a tapered form with the apex on the side of the upper edge 8 of the dorsal member 3. With the non-elastic areas P71 and P73 thus formed, the portion without the tensile force in the hip-surrounding elastic member 12 of the ventral member 72 and the dorsal member 3 has a larger left-right width than that in the disposable diaper 1. Thus, in the disposable diaper 70, a shape of the ventral member 72 and the dorsal member 3 even more largely widening from the waist opening 6 toward the leg openings 9a and 9b on the lower side can be achieved.

In the disposable diaper 70, the hip-surrounding elastic member 12c of the ventral member 72 extends in the lower edge 76 defining the left and the right leg openings of the ventral member 72. The second leg-surrounding elastic member 79 extends in the lower edge 11 defining the left and the right leg openings of the dorsal member 3. The portions of the left and the right side edges 4a and 4b of the crotch member 4 defining the left and the right leg openings of the crotch member 4 are shorter than those in the disposable undergarment 1. Thus, the crotch elastic member 75 of the crotch member 4 of the disposable diaper 70 is also shorter. The left and the right leg openings defined by the ventral member 72, the dorsal member 3, and the crotch member 4 are smaller than those in the disposable diaper 1. Thus, the disposable diaper 70 compromises the movability of the legs to achieve better fitting and more effective leakage prevention for excursion due to the large area of the ventral member 72.

### Industrial Applicability

The present invention is suitably used for producing a short rise length pant-type disposable diaper and pad holder with a stable wearing position that do not excessively compress a waist.

### DESCRIPTION OF THE REFERENCE NUMERAL

1, 30, 60, 70: Disposable undergarment
2, 62, 72: Ventral member
2c: Center portion
3, 63: Dorsal member
3c: Center portion
4: Crotch member
5a, 5b: Bonded portion
6: Waist opening
7: Upper edge of ventral member
7a: Center portion of upper edge of ventral member
8: Upper edge of dorsal member
9a, 9b: Left and right leg opening
10, 66, 76: Lower edge of ventral member
11, 67: Lower edge of dorsal member
12: hip-surrounding elastic member
13, 68, 78: First elastic member (first leg-surrounding elastic member)
14, 69, 79: Second leg-surrounding elastic member
25b, 75b: Ventral connection elastic member
P1, P2, P3, P31, P32, P33, P61, P62, P63, P71, P72, P73: Non-elastic area
K1, K2: Positioning bonded portion
100A, 100B: Layered web

## Claims

1. A disposable undergarment (1) comprising:
a ventral member (2) and a dorsal member (3) that are bonded to each other at both bonded portions (5a, 5b) along both end portions in a waist-around direction;
a crotch member (4) that is bonded to and bridges between both center portions (2c, 3c) of the ventral member (2) and the dorsal member (3);
a waist opening (6) defined by both upper edges (7, 8) of the ventral member (2) and the dorsal member (3);
left and right leg openings (9a, 9b) defined by the ventral member (2), the dorsal member (3), and the crotch member (4); and
a first elastic member (13) that extends upward toward the upper edges (7) from lower portions of the bonded portions (5a, 5b) of the ventral member (2) to a center in the waist-around direction,
wherein the ventral member (2) has a lower edge (10) including:
both end lines (10a, 10a) substantially in parallel with the waist-around direction;
a pair of inclined lines (10b, 10b) extending upward toward a side of the upper edge (7), from the both end lines (10a, 10a) toward the center portion (2c); and
a center line (10c) connecting between the inclined lines (10b, 10b);
wherein the dorsal member (3) has a lower edge (11) including:
both end lines (11a,11a) substantially in parallel with the waist-around direction;
a pair of inclined lines (11b,11b) extending downward toward a side opposite to the upper edge (8), from the both end lines (11a,11a) toward the center portion (3c); and
a center line (11c) connecting between the inclined lines (11b,11b),
wherein a hip-surrounding elastic member (12a to 12c) extending in the waist-around direction in the ventral member (2) has higher tensile force than a hip-surrounding elastic member (12d to 12f) extending in the waist-around direction in the dorsal member (3);
wherein the disposable undergarment (1) has a shape widening from the waist opening (6) toward the left and the right leg openings (9a, 9b) on a lower side, and
wherein the upper edge (7) of the ventral member (2) has a center portion (7a) disposed lower than the upper edge (8) of the dorsal member (3).

2. The disposable undergarment (1) according to claim 1, wherein a non-elastic area (P1) in which the elastic member (12, 13) loses tensile force is formed at a center of the ventral member (2) in the waist-around direction to have a tapered shape with an apex on a side of the upper edge (7).

3. The disposable undergarment (1) according to claim 2, wherein the non-elastic area (P3) in which the elastic member (12) loses the tensile force is formed at a center of the dorsal member (3) in the waist-around direction to have the tapered shape with the apex on the side of the upper edge (8).

4. The disposable undergarment (1) according to claim 1,
wherein the crotch member (4) includes at least one pair of ventral connection elastic members (25b),
wherein ventral end portions of portions where the ventral connection elastic members (25b) are bonded to the crotch member (4) overlap with areas of the crotch member (4) bonded on the ventral member (2) in an overlapping manner, and
wherein dorsal end portions of portions where the ventral connection elastic members (25b) are bonded to the crotch member (4) do not overlap with areas of the crotch member (4) bonded on the dorsal member (3) in an overlapping manner.

5. The disposable undergarment (1) according to claim 1, wherein in a state where the ventral member (2) and the dorsal member (3) are separated from each other and stretched, a lower edge (10) of the ventral member (2) and a lower edge (11) of the dorsal member (3) match upon being brought into contact with each other.

6. The disposable undergarment (1) according to claim 1, wherein positioning bonded portions (K1, K2) with which the ventral member (2) and the dorsal member (3) are partially bonded to each other are formed only around the lower edges (10, 11) of both the ventral member (2) and the dorsal member (3) to partially overlap the bonded portions (5a, 5b) or to be in peripheries of the bonded portions (5a, 5b).

7. A method for manufacturing the disposable undergarment (1) according to claim 1, the method comprising:
arranging a layered web (100A) including a series of the ventral members (2) arranged on a pitch-to-pitch basis in a flow direction (MD) and a layered web (100B) including a series of the dorsal members (3) arranged on the pitch-to-pitch basis in the flow direction (MD) in parallel with each other with lower edges (10) of the ventral members (2) and lower edges (11) of the dorsal members (3) facing each other while being separated from each other;
bonding the crotch member (4) in each pitch in the flow direction (MD), the crotch member (4) bridging between the two layered webs (100A, 100B) arranged in parallel with each other while being separated from each other;
folding the crotch member (4) in two in such a manner that the webs (100A, 100B) are overlapped with each other, while the lower edges (10a) of both end portions (2a,2b) of the ventral member (2) of the web (100A) are positioned to be aligned with the lower edges (11a) of both end portions (3a,3b) of the dorsal member (3) of the web (100B) in each pitch in the flow direction (MD) and the upper edge (7) of both end portions (2a,2b) of the ventral member (2) is not aligned with the upper edge (8) of both end portions (3a,3b) of the dorsal member (3);
forming positioning bonded portions (K1, K2) with which the both webs (100A, 100B) are partially bonded to each other, only around the lower edge (10) of the ventral member (2) and the lower edge (11) of the dorsal member (3) of the both webs (100A, 100B) overlapped with each other, to partially overlap the bonded portions (5a, 5b) or to be in peripheries of the bonded portions (5a, 5b);
stretching the upper edge (7) of the ventral member (2) of the web (100A) toward the upper edge (8) of the dorsal member (3) of the web (100B), and positioning the upper edge (7) of the ventral member (2) to be aligned with the upper edge (8) of the dorsal member (3);
bonding the both webs (100A, 100B) along a width direction (CD) in each pitch in the flow direction (MD); and
cutting the both webs (100A, 100B) along the bonded portion (5a, 5b).

## Patentansprüche

1. Einwegunterwäsche (1), umfassend:
ein ventrales Element (2) und ein dorsales Element (3), die miteinander an beiden verbundenen Abschnitten (5a, 5b) entlang beider Endabschnitte in einer Taillenumlaufrichtung miteinander verbunden sind;
ein Schrittelement (4), das mit beiden Mittelabschnitten (2c, 3c) des ventralen Elements (2) und des dorsalen Elements (3) verbunden ist und diese überbrückt;
eine Taillenöffnung (6), die durch die beiden oberen Ränder (7, 8) des ventralen Elements (2) und des dorsalen Elements (3) definiert ist;
linke und rechte Beinöffnungen (9a, 9b), die durch das ventrale Element (2), das dorsale Element (3) und das Schrittelement (4) definiert sind; und
ein erstes elastisches Element (13), das sich von unteren Abschnitten der verbundenen Abschnitte (5a, 5b) des ventralen Elements (2) nach oben in Richtung der oberen Ränder (7) zu einer Mitte in der Richtung um die Taille erstreckt,
wobei das ventrale Element (2) einen unteren Rand (10) aufweist, enthaltend:
die beiden Endlinien (10a, 10a) im Wesentlichen parallel zur Taillenumlaufrichtung;
ein Paar geneigter Linien (10b, 10b), die sich von den beiden Endlinien (10a, 10a) nach oben zu einer Seite des oberen Randes (7) in Richtung des Mittelabschnitts (2c) erstrecken; und
eine Mittellinie (10c), die zwischen den schrägen Linien (10b, 10b) verbindet;
wobei das dorsale Element (3) einen unteren Rand (11) aufweist,
enthaltend:
beide Endlinien (11a,11a) im Wesentlichen parallel zur Taillenumlaufrichtung;
ein Paar geneigter Linien (11b, 11b), die sich von den beiden Endlinien (11a, 11a) nach unten zu einer des oberen Rands (8) gegenüberliegenden Seite in Richtung des Mittelabschnitts (3c) erstrecken; und
eine Mittellinie (11c), die zwischen den schrägen Linien (11b, 11b) verbindet,
wobei ein hüftumschließendes elastisches Element (12a bis 12c), das sich in der Taillenumlaufrichtung in dem ventralen Element (2) erstreckt, eine höhere Zugkraft aufweist als ein hüftumschließendes elastisches Element (12d bis 12f), das sich in der Taillenumlaufrichtung in dem dorsalen Element (3) erstreckt;
wobei die Einwegunterwäsche (1) eine Form aufweist, die sich von der Taillenöffnung (6) in Richtung der linken und rechten Beinöffnung (9a, 9b) an einer unteren Seite verbreitert, und
wobei der obere Rand (7) des ventralen Elements (2) einen Mittelabschnitt (7a) aufweist, der niedriger angeordnet ist als der obere Rand (8) des dorsalen Elements (3).

2. Einwegunterwäsche (1) nach Anspruch 1, wobei ein nicht-elastischer Bereich (P1), in dem das elastische Element (12, 13) an Zugkraft verliert, in einer Mitte des ventralen Elements (2) in Taillenumlaufrichtung ausgebildet ist, um eine verjüngte Form mit einem Scheitelpunkt auf einer Seite des oberen Rands (7) aufzuweisen.

3. Einwegunterwäsche (1) nach Anspruch 2, wobei der nicht-elastische Bereich (P3), in dem das elastische Element (12) die Zugkraft verliert, in einer Mitte des dorsalen Elements (3) in Taillenumlaufrichtung ausgebildet ist, um die verjüngte Form mit dem Scheitelpunkt auf der Seite dem oberen Rand (8) aufzuweisen.

4. Einwegunterwäsche (1) nach Anspruch 1,
wobei das Schrittelement (4) mindestens ein Paar ventraler elastischer Verbindungselemente (25b) aufweist,
wobei ventrale Endabschnitte von Bereichen, in denen die ventralen elastischen Verbindungselemente (25b) mit dem Schrittelement (4) verbunden sind, mit Bereichen des Schrittelements (4), die mit dem ventralen Element (2) überlappend verbunden sind, überlappen und
wobei dorsale Endabschnitte von Bereichen, an denen die ventralen elastischen Verbindungselemente (25b) mit dem Schrittelement (4) verbunden sind, nicht mit Bereichen des Schrittelements (4), die auf dem dorsalen Glied (3) überlappend verbunden sind, überlappen.

5. Einwegunterwäsche (1) nach Anspruch 1, wobei in einem Zustand, in dem das ventrale Element (2) und das dorsale Element (3) voneinander getrennt und gedehnt sind, ein unterer Rand (10) des ventralen Elements (2) und ein unterer Rand (11) des dorsalen Elements (3) zusammenpassen, nachdem sie in Kontakt miteinander gebracht worden sind.

6. Einwegunterwäsche (1) nach Anspruch 1, wobei Positionierungs-Verbindungsabschnitte (K1, K2), mit denen das ventrale Element (2) und das dorsale Element (3) teilweise miteinander verbunden sind, nur um die unteren Ränder (10, 11) sowohl des ventralen Elements (2) als auch des dorsalen Elements (3) herum ausgebildet sind, um die Verbindungsabschnitte (5a, 5b) teilweise zu überlappen oder um sich in den Randbereichen der Verbindungsabschnitte (5a, 5b) zu befinden.

7. Verfahren zur Herstellung der Einwegunterwäsche (1) nach Anspruch 1, wobei das Verfahren umfasst:
Anordnen einer geschichteten Bahn (100A), die eine Reihe der ventralen Elemente (2) enthält, die auf einer Abstand-zu-Abstandbasis in einer Fließrichtung (MD) angeordnet sind, und einer geschichteten Bahn (100B), die eine Reihe der dorsalen Elemente (3) enthält, die auf der Abstand-zu-Abstandbasis in der Fließrichtung (MD) parallel zueinander angeordnet sind, wobei untere Ränder (10) der ventralen Elemente (2) und untere Ränder (11) der dorsalen Elemente (3) einander zugewandt sind, während sie voneinander getrennt sind;
Verkleben des Schrittelements (4) in jedem Abstand in Fließrichtung (MD), wobei das Schrittelement (4) zwischen den beiden parallel zueinander angeordneten, voneinander getrennten geschichteten Bahnen (100A, 100B) überbrückt;
Falten des Schrittelements (4) in zwei derart, dass die Bahnen (100A, 100B) einander überlappen, während die unteren Ränder (10a) der beiden Endabschnitte (2a, 2b) des ventralen Elements (2) der Bahn (100A) so positioniert sind, dass sie mit den unteren Rändern (11a) der beiden Endabschnitte (3a,3b) des dorsalen Elements (3) der Bahn (100B) in jeder Teilung in der Fließrichtung (MD) ausgerichtet sind und der obere Rand (7) beider Endabschnitte (2a, 2b) des ventralen Elements (2) nicht mit dem oberen Rand (8) der beiden Endabschnitte (3a, 3b) des dorsalen Elements (3) ausgerichtet ist;
Ausbilden von Positionierungs-Verbindungsabschnitten (K1, K2), mit denen die beiden Bahnen (100A, 100B) teilweise miteinander verbunden sind, nur um den unteren Rand (10) des ventralen Elements (2) und den unteren Rand (11) des dorsalen Elements (3) der beiden Bahnen (100A, 100B) miteinander zu überlappen, um die Verbindungsabschnitte (5a, 5b) teilweise zu überlappen oder um in den Randbereichen der Verbindungsabschnitte (5a, 5b) zu sein;
Strecken des oberen Rands (7) des ventralen Elements (2) der Bahn (100A) in Richtung des oberen Rands (8) des dorsalen Elements (3) der Bahn (100B) und Positionieren des oberen Rands (7) des ventralen Elements (2), so dass er mit dem oberen Rand (8) des dorsalen Elements (3) ausgerichtet ist;
Verkleben der beiden Bahnen (100A, 100B) entlang einer Breitenrichtung (CD) in jedem Abstand in Fließrichtung (MD); und
Schneiden der beiden Bahnen (100A, 100B) entlang des verbundenen Abschnitts (5a, 5b).

## Revendications

1. Sous-vêtement jetable (1) comprenant :
un élément ventral (2) et un élément dorsal (3) qui sont liés l'un à l'autre au niveau des deux parties liées (5a, 5b) le long des deux parties d'extrémité dans une direction autour de la taille ;
un élément entrejambe (4) qui est lié aux deux parties centrales et forme un pont entre les deux parties centrales (2c, 3c) de l'élément ventral (2) et de l'élément dorsal (3) ;
une ouverture de taille (6) définie par les deux bords supérieurs (7, 8) de l'élément ventral (2) et de l'élément dorsal (3) ;
des ouvertures de jambe gauche et droite (9a, 9b) définies par l'élément ventral (2), l'élément dorsal (3), et l'élément entrejambe (4) ; et
un premier élément élastique (13) qui s'étend vers le haut en direction des bords supérieurs (7) à partir de parties inférieures des parties liées (5a, 5b) de l'élément ventral (2) jusqu'à un centre dans la direction autour de la taille,
dans lequel l'élément ventral (2) a un bord inférieur (10) comportant :
les deux lignes d'extrémité (10a, 10a) sensiblement parallèles à la direction autour de la taille ;
une paire de lignes inclinées (10b, 10b) s'étendant vers le haut en direction d'un côté du bord supérieur (7), à partir des deux lignes d'extrémité (10a, 10a) en direction de la partie centrale (2c) ; et
une ligne centrale (10c) se raccordant entre les lignes inclinées (10b, 10b) ;
dans lequel l'élément dorsal (3) a un bord inférieur (11) comportant :
les deux lignes d'extrémité (11a, 11a) sensiblement parallèles à la direction autour de la taille ;
une paire de lignes inclinées (11b, 11b) s'étendant vers le bas en direction d'un côté opposé au bord supérieur (8), à partir des deux lignes d'extrémité (11a, 11a) en direction de la partie centrale (3c) ; et
une ligne centrale (11c) se raccordant entre les lignes inclinées (11b, 11b),
dans lequel un élément élastique entourant les hanches (12a à 12c) s'étendant dans la direction autour de la taille dans l'élément ventral (2) a une plus grande force de traction qu'un élément élastique entourant les hanches (12d à 12f) s'étendant dans la direction autour de la taille d'un élément dorsal (3) ;
dans lequel le sous-vêtement jetable (1) à une forme s'élargissant à partir de l'ouverture de taille (6) en direction des ouvertures de jambe gauche et droite (9a, 9b) sur un côté inférieur, et
dans lequel le bord supérieur (7) de l'élément ventral (2) à une partie centrale (7a) disposée plus basse que le bord supérieur (8) de l'élément dorsal (3).

2. Sous-vêtement jetable (1) selon la revendication 1, dans lequel une zone non élastique (P1) dans laquelle l'élément élastique (12, 13) perd une force de traction est formée au niveau d'un centre de l'élément ventral (2) dans la direction autour de la taille pour avoir une forme effilée avec un sommet sur un côté du bord supérieur (7).

3. Sous-vêtement jetable (1) selon la revendication 2, dans lequel la zone non élastique (P3) dans laquelle l'élément élastique (12) perd la force de traction est formée au niveau d'un centre de l'élément dorsal (3) dans la direction autour de la taille pour avoir la forme effilée avec le sommet sur le côté du bord supérieur (8).

4. Sous-vêtement jetable (1) selon la revendication 1,
dans lequel l'élément d'entrejambe (4) comporte au moins une paire d'éléments élastiques de raccordement ventraux (25b),
dans lequel des parties d'extrémité ventrales de parties où les éléments élastiques de raccordement ventraux (25b) sont liés à l'élément d'entrejambe (4) chevauchent des zones de l'élément d'entrejambe (4) liées sur l'élément ventral (2) d'une manière chevauchante, et
dans lequel des parties d'extrémité dorsales de parties où les éléments élastiques de raccordement ventraux (25b) sont liés à l'élément d'entrejambe (4) ne chevauchent pas de zones de l'élément d'entrejambe (4) liées sur l'élément dorsal (3) d'une manière chevauchante.

5. Sous-vêtement jetable (1) selon la revendication 1, dans lequel dans un état où l'élément ventral (2) et l'élément dorsal (3) sont séparés l'un de l'autre et étirés, un bord inférieur (10) de l'élément ventral (2) et un bord inférieur (11) de l'élément dorsal (3) se correspondent lorsqu'ils sont mis en contact l'un avec l'autre.

6. Sous-vêtement jetable (1) selon la revendication 1, dans lequel des parties liées de positionnement (K1, K2) avec lesquelles l'élément ventral (2) et l'élément dorsal (3) sont partiellement liés l'un à l'autre sont formées uniquement autour des bords inférieurs (10, 11) de l'élément ventral (2) et de l'élément dorsal (3) pour partiellement chevaucher les parties liées (5a, 5b) ou pour être dans des périphéries des parties liées (5a, 5b).

7. Procédé de fabrication du sous-vêtement jetable (1) selon la revendication 1, le procédé comprenant :
l'agencement d'une bande stratifiée (100A) comprenant une série des éléments ventraux (2) agencés sur une base pas-à-pas dans une direction de flux (MD) et une bande stratifiée (100B) comprenant une série des éléments dorsaux (3) agencés sur la base pas-à-pas dans la direction de flux (MD) parallèles les uns aux autres avec les bords inférieurs (10) des éléments ventraux (2) et les bords inférieurs (11) des éléments dorsaux (3) se faisant face les uns les autres alors qu'ils sont séparés les uns des autres ;
la liaison de l'élément d'entrejambe (4) dans chaque pas dans la direction de flux (MD), l'élément d'entrejambe (4) formant un pont entre les deux bandes stratifiées (100A, 100B) agencées parallèlement l'une à l'autre alors qu'elles sont séparées l'une de l'autre ;
le pliage de l'élément d'entrejambe (4) en deux de telle manière que les bandes (100A, 100B) se chevauchent l'une et l'autre, alors que les bords inférieurs (10a) des deux parties d'extrémité (2a, 2b) de l'élément ventral (2) de la bande (100A) sont positionnés pour être alignés avec les bords inférieurs (11a) des deux parties d'extrémité (3a, 3b) de l'élément dorsal (3) de la bande (100B) dans chaque pas dans la direction de flux (MD) et le bord supérieur (7) des deux parties d'extrémité (2a, 2b) de l'élément ventral (2) n'est pas aligné avec le bord supérieur (8) des deux parties d'extrémité (3a, 3b) de l'élément dorsal (3) ;
la formation de parties liées de positionnement (K1, K2) avec lesquelles les deux bandes (100A, 100B) sont partiellement liées l'une à l'autre, uniquement autour du bord inférieur (10) de l'élément ventral (2) et du bord inférieur (11) de l'élément dorsal (3) les deux bandes (100A, 100B) se chevauchant l'une l'autre, pour partiellement chevaucher les parties liées (5a, 5b) ou pour être dans des périphéries des parties liées (5a, 5b) ;
l'étirement du bord supérieur (7) de l'élément ventral (2) de la bande (100A) en direction du bord supérieur (8) de l'élément dorsal (3) de la bande (100B), et le positionnement du bord supérieur (7) de l'élément ventral (2) en alignement avec le bord supérieur (8) de l'élément dorsal (3) ;
la liaison des deux bandes (100A, 100B) le long d'une direction de la largeur (CD) dans chaque pas dans la direction de flux (MD) ; et
la découpe des deux bandes (100A, 100B) le long de la partie liée (5a, 5b).
